# EUROPÄISCHE PATENTANMELDUNG

(11) **EP 4 706 544 A2**
(43) Veröffentlichungstag der Anmeldung: **11.03.2026**
(21) Anmeldenummer: 26152556.2
(22) Anmeldetag: 29.02.2024
(51) Int. Cl.: A61B 6/03, H04B 7/00, A61B 6/00

(54) **VERFAHREN ZUR DURCHFÜHRUNG EINER DATENÜBERTRAGUNG VON EINEM ROTIERENDEN ABSCHNITT EINES MEDIZINISCHEN BILDGEBUNGSGERÄTS ZU EINEM STATIONÄREN ABSCHNITT DES MEDIZINISCHEN BILDGEBUNGSGERÄTS UND MEDIZINISCHE BILDGEBUNGSEINRICHTUNG**

(62) Teilanmeldung aus: 24160420.6
(71) Anmelder: Siemens Healthineers AG, 91301 Forchheim (DE)
(72) Erfinder: Hohl, Christoph, 91058 Erlangen (DE); Hosemann, Michael, 91301 Forchheim (DE); Düppenbecker, Peter Michael, 91058 Erlangen (DE)
(74) Vertreter: Siemens Healthineers Patent Attorneys

(57) **Zusammenfassung**

Die Erfindung betrifft ein Verfahren zur Durchführung einer Datenübertragung von einem rotierenden Abschnitt (4) eines medizinischen Bildgebungsgeräts (2) zu einem stationären Abschnitt (3) des medizinischen Bildgebungsgeräts (2), wobei die Datenübertragung mittels an dem rotierenden Abschnitt (4) angeordneten Funksendern (11, 13, 17, 18) und an dem stationären Abschnitt (3) angeordneten Funkempfängern (12, 15, 19, 20) erfolgt, wobei mehrere separate Übertragungspaare, die jeweils einen der Funksender (11) und einen der Funkempfänger (12) umfassen, gebildet werden, wobei bei den Übertragungspaaren jeweils Funksignale von dem Funksender (11) zu dem Funkempfänger (12) übertragen werden und eine die Funksignale betreffende Hauptabstrahlrichtung des Funksenders (11) zu der Richtung des Funkempfängers (12) nachgeführt wird.

## Beschreibung

Die vorliegende Erfindung betrifft ein Verfahren zur Durchführung einer Datenübertragung von einem rotierenden Abschnitt eines medizinischen Bildgebungsgeräts zu einem stationären Abschnitt des medizinischen Bildgebungsgeräts, wobei die Datenübertragung mittels an dem rotierenden Abschnitt angeordneten Funksendern und an dem stationären Abschnitt angeordneten Funkempfängern erfolgt.

Medizinische Bildgebungsgeräte, etwa Computertomographiegeräte, die oft als CT-geräte abgekürzt werden, weisen häufig einen rotierenden und einen stationären Abschnitt auf. Der stationäre Abschnitt ist bezüglich einer Umgebung des Bildgebungsgeräts ortsfest angeordnet. Wenn sich das Bildgebungsgerät in einem Rotationszustand befindet, in dem etwa Daten betreffend ein mittels des Bildgebungsgeräts zu erzeugendes Bild erfasst werden, rotiert respektive dreht sich der rotierende Abschnitt relativ zu dem ortsfesten Abschnitt. Diese Rotation erfolgt um eine bezüglich des stationären Abschnitts feste Rotationsachse, die typischerweise eine Systemachse des Bildgebungsgeräts bildet.

In dem Rotationszustand fallen seitens des rotierenden Abschnitts häufig große Datenmengen an, die zur Weiterverarbeitung an den stationären Abschnitt übertragen werden müssen. Diese Übertragung hat typischerweise möglichst zeitnah zu erfolgen, etwa unmittelbar nach der Generierung der Daten. Oft erlaubt die nicht unerhebliche Menge anfallender Daten keine Zwischenspeicherung selbiger seitens des rotierenden Abschnitts, sodass die unmittelbare Übertragung alternativlos ist. Dieses Problem wird in Zukunft zunehmen, insbesondere da aufgrund höherer Orts- sowie Energieauflösungen bei modernen Bildgebungsverfahren immer größere Datenmengen anfallen.

Häufig erfolgt die Datenübertragung über ein Schleifringsystem, bei dem ein elektrischer Schleifkontakt eine die Datenübertragung ermöglichende Kopplung zwischen dem rotierenden Abschnitt und dem stationären Abschnitt herstellt. Allerdings stößt dieses Konzept häufig an seine Grenzen, und zwar wenn eine aktuell erforderliche Datenübertragungsrate die mittels des Schleifringsystems maximal realisierbare Datenübertragungsrate übersteigt. Ein weiterer Nachteil ist, dass Verschleißeffekte und/oder Verschmutzungen Beeinträchtigungen bezüglich der Datenübertrag über den Schleifkontakt bewirken können.

In US 2016 / 0 256 129 A1 ist eine mögliche Lösung dieser Problematiken beschrieben. So werden bei dem dort offenbarten Computertomografiegerät Daten von einem rotierenden Part zu einem stationären Part per Funk übertragen.

Die vorliegende Erfindung stellt sich die Aufgabe, eine Weiterbildung des Konzepts der funkbasierten Datenübertragung von einem rotierenden Abschnitt zu einem stationären Abschnitt bei einem medizinischen Bildgebungsgerät anzugeben, insbesondere hinsichtlich einer hohen Datenübertragungsrate.

Gemäß der vorliegenden Erfindung wird diese Aufgabe im Rahmen dreier erfindungsgemäßer Ausführungen gelöst, die nachfolgend nacheinander erläutert werden. Die für eine der Ausführungen erläuterten Vorteile, Merkmale und Aspekte sind auch auf die anderen Ausführungen übertragbar, sofern diese nicht ausdrücklich und in unvereinbarer Weise voneinander abweichen.

Gemäß der ersten erfindungsgemäßen Ausführung wird das der vorliegenden Erfindung zugrunde liegende Problem bei einem Verfahren der eingangs genannten Art dadurch gelöst, dass mehrere separate Übertragungspaare, die jeweils einen der Funksender und einen der Funkempfänger umfassen, gebildet werden, wobei bei den Übertragungspaaren jeweils Funksignale von dem Funksender zu dem Funkempfänger übertragen werden und eine die Funksignale betreffende Hauptabstrahlrichtung des Funksenders zu der Richtung des Funkempfängers nachgeführt wird.

Die Erfindung beruht hinsichtlich dieser erfindungsgemäßen Ausführung mitunter auf dem Gedanken, dass Pausen bei der Datenübertragungspausen vermieden werden. So würde sich der Funkempfänger aus der Sicht des jeweiligen, auf dem rotierenden Abschnitt angeordneten Funksenders aufgrund der Rotation zwangsläufig aus der Hauptabstrahlrichtung dieses Funksenders bewegen. Um dies zu vermeiden ist die Hauptabstrahlrichtung des Funksenders relativ zu dem rotierenden Abschnitt veränderbar, sodass die scheinbare Änderung der Position des Funkempfängers mittels der Nachführung der Hauptabstrahlrichtung ausgeglichen wird und hierdurch die Übertragung der Funksignale aufrechterhalten werden kann. Unter der Hauptabstrahlrichtung wird eine Raumrichtung verstanden, zu der hin die Funksignale respektive die die Funksignale bildenden elektromagnetischen Wellen mit der im Vergleich zu den übrigen Raumrichtungen größten Intensität emittiert werden. Im Extremfall erfolgt die Emittierung der Funksignale ausschließlich in die Hauptabstrahlrichtung.

Angenommen, die Hauptabstrahlrichtung wäre fest, dann würde diese zusammen mit dem rotierenden Abschnitt eine synchrone Rotation ausführen. Bezogen auf das Übertragungspaar hätte dies zur Folge, dass eine Übertragung von Funksignalen von dem jeweiligen Funksender zu dem jeweiligen Funkempfänger nur in denjenigen Momenten erfolgen könnte, in denen die Hauptabstrahlrichtung die Position des Funkempfängers trifft. Die Übertragung von Funksignalen wäre bei diesem Übertragungspaar nur zu diskreten Zeitpunkten oder innerhalb enger Zeitfenster möglich, was die Datenübertragungsrate stark limitieren würde. Durch die erfindungsgemäß vorgesehene Nachführung der Hauptabstrahlrichtung wird es ermöglicht, dass diese Zeitfenster vergrößert werden.

Die Funksender können jeweils eine Antenne aufweisen, mittels der die Funksignale generierbar sind. Mittels Steuersignalen können in der Antenne bewegte Ladungsträger erzeugt werden, die wiederum die Generierung der Funksignale bewirken. Die Abstrahlintensität folgt hierbei typischerweise einer keulenförmigen Verteilung, wobei die Hauptabstrahlrichtung einer Zentralachse der Keule oder einer der Keulen entspricht. Die Steuersignale können mittels einer mit den Funksendern verbundenen Steuerungseinrichtung generiert werden. Die Steuerungseinrichtung kann eine Komponente des medizinischen Bildgebungsgeräts. Das medizinische Bildgebungsgerät und die Steuerungseinrichtung können eine medizinische Bildgebungseinrichtung bilden.

Die Funkempfänger können jeweils eine Antenne aufweisen, mittels der die Funksignale detektiert werden. Auf die Antenne auftreffende elektromagnetische Wellen respektive Funksignale erzeugen in der Antenne bewegte Ladungsträger, was wiederum die Detektion dieser elektromagnetischen Wellen respektive Funksignale ermöglicht, insbesondere vermittels der mit den Funkempfängern verbundenen Steuereinrichtung.

Insbesondere da deren gegenständliche Ausgestaltungen ähnlich oder sogar identisch sein und sich nur hinsichtlich ihrer Ansteuerung unterscheiden können, kann wenigstens einer der Funksender grundsätzlich auch als ein Empfänger für Funksignale verwendet werden und umgekehrt, sodass auch eine Datenübertragung von dem stationären Abschnitt zu dem rotierenden Abschnitt ermöglicht wird, beispielsweise zur Übertragung von Steuerdaten respektive -befehlen. So ist im Rahmen der vorliegenden Erfindung neben einer unidirektionalen Datenübertragung von dem rotierenden Abschnitt zu dem stationären Abschnitt auch eine bidirektionale Datenübertragung denkbar. Zudem oder alternativ kann hierfür auch ein Schleifringsystem gemäß dem eingangs bereits Erläuterten vorgesehen sein.

Die Funksender können fest, das heißt unbeweglich, an dem rotierenden Abschnitt angeordnet sein. Die Änderung der Hauptabstrahlrichtung kann mechanisch und/oder elektronisch erfolgen. Bezüglich der mechanischen Änderung ist denkbar, dass wenigstens einer der Funksender einen an dem rotierenden Abschnitt fest montierten, festen Abschnitt und einen über ein Gelenk, insbesondere ein Schwenkgelenk, mit dem festen Abschnitt verbundenen, beweglichen Abschnitt aufweist. Ein etwa mittels der Steuerungseinrichtung ansteuerbarer, insbesondere elektromechanischer, Aktor kann eine Relativbewegung des beweglichen Abschnitts relativ zu dem festen Abschnitt bewirken, sodass die seitens des beweglichen Abschnitts generierten Funksignale ihre Hauptabstrahlrichtung ändern. Im Rahmen dieser Ausführung wird eine mechanisch verschwenkbare Antenne realisiert.

Bezüglich der elektronischen Änderung der Hauptabstrahlrichtung ist denkbar, dass wenigstens einer der Funkempfänger eine Beamforming-Antenne aufweist oder ist. Diese kann eine Phased-Array-Antenne realisieren, die mehrere individuell ansteuerbare Einzelantennen umfasst, wobei eine Ansteuerung der Einzelantennen, etwa mittels der Steuerungseinrichtung, eine Einstellung einer Richtwirkung und mithin der Hauptabstrahlrichtung bewirkt. In diesem Zusammenhang wird oft von einer elektronischen Verschwenkbarkeit der Antenne gesprochen.

An dem rotierenden Abschnitt können mit den Funksendern verbundene Sensoren zur Erfassung von Daten betreffend ein mittels des Bildgebungsgeräts zu erzeugenden Bildes angeordnet sein. Die Datenübertragung von dem rotierenden Abschnitt zu dem stationären Abschnitt dient in erster Linie der Übertragung dieser Daten. Die Sensoren können jeweils wenigstens ein Detektor sein oder einen solchen umfassen, etwa einen Röntgendetektor mit beispielsweise einem Szintillator.

Denkbar ist, dass wenigstens einer der Funksender und wenigstens einer der Sensoren als ein gemeinsames Modul realisiert sind, das an dem rotierenden Abschnitt angeordnet ist. Das Modul kann einen Träger, etwa eine Leiterplatte, aufweisen, auf, an und/oder in dem die Komponenten, die den Funksender und den jeweiligen Sensor realisieren, angeordnet sind. Der jeweilige Funksender wird zur Übertragung der mittels des Sensors dieses Moduls generierten Daten verwendet.

Zur Realisierung der Datenübertragung wird zumindest ein Übertragungspaar gebildet, das einen der Funksender und einen der Funkempfänger umfasst. Jeder der Funksender und jeder der Funkempfänger ist hierbei genau oder höchstens einem Übertragungspaar zugeordnet. Bevorzugt sind die Zahl der Funksender, die Zahl der Funkempfänger und die Zahl der Übertragungspaare gleich, insbesondere während der gesamten Dauer der Durchführung der Datenübertragung.

Bei den Übertragungspaaren wird jeweils ein Übertragungskanal gebildet, über den die Funksignale übertragen werden. Die Anzahl vorhandener und die Datenübertragung realisierender Übertragungskanäle entspricht mithin maximal der Anzahl der Übertragungspaare. Die vorbeschriebene Nachführung der Hauptabstrahlrichtung kann bei allen Übertragungspaaren erfolgen.

Gemäß einer denkbaren Weiterbildung der Erfindung ist vorgesehen, dass die Übertragungspaare zu Beginn der Datenübertragung dadurch vorgegeben und/oder während der Durchführung der Datenübertragung dadurch aktualisiert werden, dass dem Funksender des jeweiligen Übertragungspaars derjenige der Funkempfänger zugeordnet wird, der aktuell den kürzesten Abstand zu diesem Funksender aufweist und/oder der mit dem jeweiligen Funksender aktuell über eine direkte Sichtlinie verbindbar ist. Im Rahmen dieser Ausführungsform erfolgt bezüglich der Vorgabe der Übertragungspaare keine beliebige Zuordnung zwischen den Funksendern und den Funkempfängern. Stattdessen wird hierzu eine spezifischen Zuordnungsvorschrift benutzt. So werden die Übertragungspaare derart gebildet, dass über diese nicht nur eine Datenübertragung grundsätzlich möglich ist, sondern dass diese auch mit der möglichst hohen Übertragungsqualität erfolgen kann.

Ersichtlich ist dies etwa für Übertragungspaare, deren Partner einen möglichst geringen Abstand zueinander aufweisen, der Fall. So nimmt die Intensität respektive die Signalstärke der die Funksignale darstellenden elektromagnetischen Wellen aufgrund der Freiraumdämpfung mit der Zunahme der Länge des Übertragungswegs ab, sodass der Übertragungsweg zur Realisierung der möglichst hohen Übertragungsqualität möglichst kurz sein sollte. Weiterhin ist diesbezüglich von Vorteil, wenn der jeweilige Funksender und der jeweilige Funkempfänger über eine unmittelbare und direkte Sichtlinie miteinander verbindbar sind, sodass die Funksingale reflexionsfrei und mithin entlang einem gänzlich geraden und knickfreien Übertragungsweg übertragen werden. Obgleich die Datenübertragung grundsätzlich auch dann denkbar ist, wenn die Funksignale auf ihrem Übertragungsweg reflektiert werden, etwa an dem rotierenden Abschnitt und/oder dem stationären Abschnitt, würde dies die Nachführung entsprechend verkomplizieren und potentiell zu einer Beeinträchtigung der Übertragungsqualität führen.

Die Zuordnung der Funksender und der Funkempfänger zu den Übertragungspaaren erfolgt zu Beginn der Datenübertragung, sodass diesbezüglich auch von einer erstmaligen Zuordnung gesprochen werden kann. Zudem verursacht auch die rotationsbedingt auftretende Änderung der Relativpositionen zwischen den Funksendern und den Funkempfängern das Erfordernis, dass im Verlauf der weiteren Durchführung der Datenübertragung Aktualisierungen respektive Neuzuordnungen der Funksender und der Funkempfänger zu den Übertragungspaaren erforderlich werden.

Im Zuge der Aktualisierung der Übertragungspaare ist es erforderlich, dass die Hauptabstrahlrichtung des jeweiligen Funksenders von der Richtung des diesem Funksender vor der Aktualisierung zugeordneten Funkempfängers zu der Richtung des diesem Funksender nach der Aktualisierung zugeordneten Funkempfängers geändert wird. So kann in einem ersten Schritt die Übertragung der Funksignale von dem Funksender zu dem Funkempfänger des vor der Aktualisierung vorliegenden Übertragungspaars beendet werden. Im zweiten Schritt kann die Hauptabstrahlrichtung geändert, etwa verschwenkt, werden. Im dritten Schritt kann die Übertragung der Funksignale von dem Funksender zu dem Funkempfänger des nach der Aktualisierung vorliegenden Übertragungspaars gestartet werden.

Denkbar ist, dass wenigstens ein die Qualität der Übertragung der Funksignale bei dem jeweiligen Übertragungspaar betreffender Qualitätsparameter ermittelt wird, wobei ein Aktualisierungszeitpunkt, an dem die oder eine Aktualisierung des wenigstens einen Übertragungspaars erfolgt, anhand des wenigstens einen Qualitätsparameters vorgegeben wird. Die Feststellung des Erfordernisses der Aktualisierung respektive die Bestimmung des Aktualisierungszeitpunkts erfolgt gemäß dieser Ausführungsform unmittelbar messtechnisch.

Der Qualitätsparameter oder einer der Qualitätsparameter kann eine Intensität respektive Signalstärke der mittels des jeweiligen Funkempfängers empfangenen Funksignale betreffen. So kann die Überprüfung eines Aktualisierungskriteriums erfolgen, bei dessen Erfüllung eine Aktualisierung des jeweiligen Übertragungspaars erfolgt. Das Aktualisierungskriterium kann erfüllt sein, wenn sich aus dem Qualitätsparameter ergibt, dass die Qualität unter einen, etwa fest vorgegebenen, Qualitätsgrenzwert fällt, insbesondere wenn die Intensität unter einen Intensitätsgrenzwert fällt. In diesem Fall impliziert die niedrige Qualität, dass der Abstand zwischen dem Funksender und dem Funkempfänger derart groß geworden ist, oder dass keine direkte Sichtlinie zwischen dem Funksender und dem Funkempfänger mehr vorhanden ist, sodass eine Übertragung der Funksignale mit hinreichender Qualität nicht mehr möglich und die Aktualisierung dieses Übertragungspaars erforderlich ist.

Denkbar ist, dass zudem oder alternativ wenigstens ein die Rotation des rotierenden Abschnitts beschreibender Rotationsparameter ermittelt wird, wobei der oder ein Aktualisierungszeitpunkt, an dem die oder eine Aktualisierung des wenigstens einen Übertragungspaars erfolgt, anhand des wenigstens einen Rotationsparameters vorgegeben wird. Die Feststellung des Erfordernisses der Aktualisierung respektive die Bestimmung des Aktualisierungszeitpunkts erfolgt gemäß dieser Ausführungsform mittelbar über eine, insbesondere rechnerische, Analyse der Rotation des rotierenden Abschnitts.

Der Rotationsparameter kann eine Rotationsfrequenz der Rotation des rotierenden Abschnitts betreffen. Der oder ein oder ein weiterer Rotationsparameter kann eine Phase der Rotation des rotierenden Abschnitts betreffen. So können, etwa mittels der Steuerungseinrichtung, Steuersignale zur Steuerung der Rotation des rotierenden Abschnitts generiert und an den rotierenden Abschnitt respektive einen mit dem rotierenden Abschnitt verbundenen Elektromotor übertragen werden. Als Steuerbasis zur Vorgabe der Steuersingale kann eine nutzerseitig vorgenommene Nutzereingabe und/oder ein fest vorgegebener Programmablauf genutzt werden, wobei anhand dieser Steuerbasis der wenigstens eine Rotationsparameter ermittelt wird.

Anhand des wenigstens einen Rotationsparameters können zeitabhängige Relativpositionen zwischen den Funksendern und den Funkempfängern bestimmt werden, wobei diese Werte als eine Grundlage für die Festlegung des Aktualisierungszeitpunkts respektive der Aktualisierungszeitpunkte genutzt werden. Hierzu können Zeitintervalle bestimmt werden, während denen eine direkte Sichtlinie zwischen dem jeweiligen Funksender und dem jeweiligen Funkempfänger vorliegt, wobei zum Ende dieses Zeitintervalls eine Aktualisierung angezeigt ist.

Bezüglich der Vorgabe des Aktualisierungszeitpunkts ist denkbar, dass sowohl der wenigstens eine Qualitätsparameter als auch der wenigstens eine Rotationsparameter genutzt werden. So kann anhand des Rotationsparameters der Aktualisierungszeitpunkt rechnerisch bestimmt und anhand des Qualitätsparameters messtechnisch verifiziert werden. Insbesondere ist denkbar, dass auch der Rotationsparameter anhand des Qualitätsparameters berichtigt, beziehungsweise aktualisiert wird. Denkbar ist, dass die Aktualisierung für wenigstens zwei der Übertragungspaare, insbesondere für alle Übertragungspaare, simultan erfolgt. So können die jeweiligen Funksender und die jeweiligen Funkempfänger derart symmetrisch, insbesondere gleichmäßig, verteilt angeordnet sein, dass das Erfordernis der Aktualisierung bei den Übertragungspaaren gleichzeitig eintritt.

Zusätzlich zu oder unabhängig von den im Zusammenhang mit der Bestimmung des Aktualisierungszeitpunkts zuvor dargelegten Aspekten ist denkbar, dass die Nachführung der Hauptabstrahlrichtung basierend auf dem wenigstens einen Qualitätsparameter und/oder dem wenigstens einen Rotationsparameter erfolgt. Anders ausgedrückt kann der wenigstens eine Qualitätsparameter und/oder der wenigstens eine Rotationsparameter als eine Steuerbasis für die Bestimmung der Aktualisierungszeitpunkte und/oder die Nachführung der Hauptabstrahlrichtung genutzt werden.

Bevorzugt bildet während eines kompletten Umlaufs des rotierenden Abschnitts jeder der Funksender mit jedem der Funkempfänger eines der Übertragungspaare. Hierbei werden die Übertragungspaare während der Rotation des rotierenden Abschnitts und mithin während der Datenübertragung derart aktualisiert, dass sukzessive alle möglichen Übertragungspaare gebildet werden, sodass während der Datenübertragung möglichst wenige, insbesondere keine, Zeitfenster entstehen, während derer keine oder nur eine reduzierte Datenübertragung möglich ist.

Gemäß der zweiten erfindungsgemäßen Ausführung wird das der vorliegenden Erfindung zugrunde liegende Problem bei einem Verfahren der eingangs genannten Art dadurch gelöst, dass mehrere separate Übertragungspaare, die jeweils einen der Funksender und einen der Funkempfänger umfassen, gebildet werden, wobei bei den Übertragungspaaren jeweils Funksignale von dem Funksender zu dem Funkempfänger übertragen werden, wobei zu den mittels der Funkempfänger empfangenen Funksignalen jeweils wenigstens eine Zuordnungsinformation ermittelt wird, die denjenigen Funksender beschreibt, der dieses Funksignal generiert hat, wobei bei den Übertragungspaaren jeweils mittels der wenigstens einen Zuordnungsinformation diejenigen von dem Funkempfänger empfangenen Funkdaten ausgefiltert werden, die nicht von dem Funksender dieses Übertragungspaars generiert wurden.

Gemäß dieser Ausführungsform erfolgt die Datenübertragung simultan respektive gleichzeitig über mehrere Übertragungskanäle, die jeweils durch eines der Übertragungspaare realisiert werden, wobei die maximal mögliche Datenübertragungsrate mit der Zahl der realisierten Übertragungskanäle korreliert. Somit erfolgt im Rahmen dieser Ausführung eine gleichzeitige Generierung von Funksignalen mittels der Funksender. Hierbei tritt das Problem auf, dass seitens der Funkempfänger jeweils Funksignale mehrerer Funksender erfasst werden. Folglich ist eine Filterung dahingehend erforderlich, dass bei jedem Übertragungspaar nur diejenigen seitens des Funkempfängers empfangenen Funksignale im Zuge des jeweiligen Übertragungskanals benutzt und weiterverwendet werden, die von dem dem jeweiligen Funkempfänger zugeordneten Funksender stammen. Zu diesem Zweck ist den Funksignalen jeweils die Zuordnungsinformation zugeordnet, die zusammen mit dem jeweiligen Funksignal erfasst wird und anhand der sich bestimmen lässt, von welchem Funksender dieses Funksignal stammt. Die Erfindung beruht hinsichtlich der zweiten erfindungsgemäßen Ausführung mitunter auf dem Gedanken, dass bei jedem der Übertragungspaare nur diejenigen empfangenen Funksignale für den jeweiligen Übertragungskanal verwendet werden, für die sich aus der Zuordnungsinformation ergibt, dass diese von dem Funksender dieses Übertragungspaars generiert wurden. Es wird somit ein sogenanntes MIMO-Konzept realisiert, wobei die Abkürzung "MIMO" für "Multiple Input Multiple Output" steht.

Die Zuordnungsinformation oder eine der Zuordnungsinformationen kann eine Distanzinformation sein, die eine Distanz zwischen dem jeweiligen Funkempfänger und dem Funksender, mittels dem das jeweilige Funksignal generiert wurde, betrifft.

So sind die Funksender an unterschiedlichen Positionen am rotierenden Abschnitt angeordnet, sodass sich auch die jeweiligen Distanzen zu den Funkempfängern unterscheiden. Dieser Umstand wird bei dieser Ausführungsform für die Zuordnung der Funksignale zu den Funksendern genutzt.

Die Distanzinformation kann eine Signalstärke respektive Intensität des jeweiligen Funksignals betreffen, wobei die Distanz mittelbar anhand der Signalstärke beschrieben wird. Falls die Übertragung von dem Funksender zu dem Funkempfänger reflexionsfrei stattfindet, dann entspricht der Übertragungsweg der direkten Sicht- beziehungsweise Verbindungslinie zwischen dem jeweiligen Funksender und dem jeweiligen Funkempfänger. Falls bei dieser Übertragung eine Reflexion auftritt, ist der Übertragungsweg entsprechend länger als die Verbindungslinie. Als die Distanzinformation kann der bereits oben erläuterte Qualitätsparameter, betreffend etwa die Intensität der Funksignale, verwendet werden.

Im Rahmen der Filterung kann vorgesehen sein, dass für jedes der Übertragungspaare die aktuelle Distanz zwischen dem Funksender und dem Funkempfänger ermittelt wird, etwa anhand des Rotationsparameters. Dieses Ergebnis kann mit der Distanzinformation abgeglichen werden, wobei eine Weiterverarbeitung respektive Nutzung des jeweiligen Funksignals nur dann erfolgt, wenn eine Übereinstimmung vorliegt.

Denkbar ist, dass die Zuordnungsinformation oder eine der Zuordnungsinformationen eine Polarisationsinformation ist, die eine Polarisation des jeweiligen Funksignals betrifft. So können mittels der Funksender jeweils Funksignale mit einer für den jeweiligen Funksender spezifischen Polarisation generiert werden. Hierzu ist eine lineare, insbesondere horizontale, vertikale oder diagonale, oder zirkuläre oder elliptische Polarisation denkbar. So kann zu den seitens der Funkempfänger erfassten Funksignalen jeweils eine Messgröße bestimmt werden, anhand der die Polarisationsinformation bestimmt wird oder die die Polarisationsinformation darstellt.

Bevorzugt sind die Polarisationen der mittels der Funksender jeweils generierten Funksignale bekannt respektive hinterlegt. Im Rahmen der Filterung kann für jedes der Übertragungspaare die Polarisation der Funksignale ermittelt beziehungsweise abgerufen werden. Diese Polarisation kann mit der Polarisationsinformation abgeglichen werden, wobei eine Weiterverarbeitung respektive Nutzung des jeweiligen Funksignals nur dann erfolgt, wenn eine Übereinstimmung vorliegt.

Grundsätzlich ist denkbar, dass die mittels der Funksender generierten Funksignale beziehungsweise die die Funksignale bildenden elektromagnetischen Wellen dieselbe Frequenz aufweisen oder zumindest im selben Frequenzband verortet sind, wobei wenigstens eine der zuvor erläuterten Informationen eine hinreichende Unterscheidbarkeit der Funksignale ermöglicht. Gleichwohl kann die Zuordnungsinformation oder eine der Zuordnungsinformationen eine Frequenzinformation sein, die eine Frequenz oder ein Frequenzband des jeweiligen Funksignals betrifft. So können mittels der Funksender jeweils Funksignale mit einer für den jeweiligen Funksender spezifischen Frequenz generiert werden. Denkbar sind diesbezüglich auch spezifische Frequenzbänder mit einer Bandbreite von beispielsweise 2 GHz. Entsprechend ist vorgesehen, dass zu den seitens der Funkempfänger erfassten Funksignalen jeweils eine Messgröße bestimmt wird, anhand der die Frequenzinformation bestimmt wird oder die die Frequenzinformation darstellt.

Bevorzugt sind die Frequenzen der mittels der Funksender jeweils generierten Funksignale bekannt respektive hinterlegt. Im Rahmen der Filterung kann vorgesehen sein, dass für jedes der Übertragungspaare die Frequenz oder das Frequenzband der jeweiligen Funksignale ermittelt, beziehungsweise abgerufen wird. Diese Frequenz oder dieses Frequenzband kann mit der Frequenzinformation abgeglichen werden, wobei eine Weiterverarbeitung respektive Nutzung des jeweiligen Funksignals nur dann erfolgt, wenn eine Übereinstimmung vorliegt.

Bezüglich der erläuterten Möglichkeiten ist vorgesehen, dass nur eine dieser Zuordnungsinformationen ermittelt und ausgewertet wird. Bevorzugt werden jedoch wenigstens zwei, besonders bevorzugt drei, dieser Zuordnungsinformationen ermittelt und ausgewertet. So kann als erste Zuordnungsinformation die Distanzinformation, als zweite Zuordnungsinformation die Polarisationsinformation und als dritte Zuordnungsinformation die Frequenzinformation vorgesehen sein.

Sofern die Zuordnungsinformation oder eine der Zuordnungsinformationen die Frequenzinformation ist, dann kann vorgesehen sein, dass die Funksender jeweils einer von mehreren Funksendergruppen zugeordnet sind, wobei die Funksender einer der Funksendergruppen jeweils Funksignale im gleichen Frequenzbereich generieren, wobei die Funksender unterschiedlicher Funksendergruppen jeweils Funksignale in verschiedenen Frequenzbereichen generieren, wobei die Funksender der Funksendergruppen jeweils gruppiert und beabstandet zu den Funksendern der wenigstens einen anderen Funksendergruppe an dem rotierenden Abschnitt angeordnet sind. Falls die Funksignale im gleichen Frequenzbereich liegen, dann weisen diese dieselbe Frequenz auf oder liegen im selben Frequenzband. Die Funksender der Funksendergruppen sind jeweils gehäuft nebeneinander angeordnet. Während die Funksender der Funksendergruppen jeweils untereinander insbesondere unmittelbar nebeneinander respektive benachbart angeordnet sind, ist zwischen den Funksendergruppen ein diesbezüglich größerer Abstand vorhanden.

Im Rahmen dieser Ausführungsform wird anhand der Frequenzinformation eine Vorfilterung hinsichtlich der Funksendergruppen ermöglicht, bevor die endgültige Filterung der Funksignale erfolgt. Aufgrund der örtlichen Nähe der Funksender der jeweiligen Funksendergruppen ist davon auszugehen, dass die Funksignale, die von diesen Funksendern generierten Funksignale hauptsächlich respektive überwiegend von Funkempfängern empfangen werden, die ebenfalls einer Funkempfängergruppe angehören, die in örtlicher Nähe zueinander an dem stationären Abschnitt angeordnet sind. Konkret können mithin auch die Funkempfänger der Funkempfängergruppen jeweils gruppiert und beabstandet zu den Funkempfängern der wenigstens einen anderen Funkempfängergruppe an dem stationären Abschnitt angeordnet sein. Die Funkempfänger der Funkempfängergruppen sind jeweils gehäuft nebeneinander angeordnet. Während die Funkempfänger der Funkempfängergruppen jeweils untereinander insbesondere unmittelbar nebeneinander respektive benachbart angeordnet sind, ist zwischen den Funkempfängergruppen ein diesbezüglich größerer Abstand vorhanden.

Die Zahl der Funksendergruppen entspricht bevorzugt der Zahl der Funkempfängergruppen. Die Zuordnungen der Funksender beziehungsweise Funkempfänger zu den Funksendergruppen beziehungsweise Funkempfängergruppen können fest sein, insbesondere wenn die Komponenten der Funksendergruppen beziehungsweise Funkempfängergruppen in örtlicher Nähe zueinander angeordnet sind. Die Übertragungspaare können derart gebildet werden, dass den Funksendern einer der Funksendergruppen nur die Funkempfänger einer der Funkempfängergruppen zugeordnet werden und umgekehrt. Mithin werden im Rahmen dieser Ausführungsform Gruppenpaare gebildet, wobei die Funksender der Funksendergruppe dieses Gruppenpaares und die Funkempfänger der Funkempfängergruppe dieses Gruppenpaares jeweils Übertragungspaare bilden.

Bevorzugt sind die Frequenzen der mittels der Funksender einer der Funksendergruppen jeweils generierten Funksignale bekannt respektive hinterlegt. Im Rahmen der Vorfilterung kann vorgesehen sein, dass für jedes der Gruppenpaare die Frequenz oder das Frequenzband der jeweiligen Funksignale bekannt ist. Diese Frequenz oder dieses Frequenzband kann mit der Frequenzinformation abgeglichen werden, wobei eine Weiterverarbeitung des jeweiligen Funksignals im Rahmen der Durchführung der Filterung nur dann erfolgt, wenn eine Übereinstimmung vorliegt. Der im Rahmen der Vorfilterung realisierte Selektionseffekt bewirkt eine weitere Erhöhung der maximal möglichen Datenübertragungsrate um einen bestimmten Faktor, ohne dass hierdurch die anschließend durchzuführende, oben beschriebene Filterung komplexer wird. Dieser Faktor entspricht der Anzahl der vorgesehenen Gruppenpaare.

Bevorzugt ist im Rahmen der zweiten erfindungsgemäßen Ausführung vorgesehen, dass die Funksender jeweils wenigstens eine bezüglich des rotierenden Abschnitts feste Hauptabstrahlrichtung der Funksignale aufweisen. Im Gegensatz zur ersten erfindungsgemäßen Ausführung ist eine Änderung der Hauptabstrahlrichtung bezüglich des rotierenden Abschnitts nicht vorgesehen. Stattdessen rotiert die Hauptabstrahlrichtung zusammen und simultan mit dem rotierenden Abschnitt. Die Hauptabstrahlrichtung der Funkempfänger kann jeweils mehrere der oder bevorzugt alle Funkempfänger nacheinander überstreichen. Die Aktualisierung der Übertragungspaare erfolgt in diesem Fall bevorzugt dahingehend, dass den Funksendern jeweils derjenige Funkempfänger zugeordnet wird, der aktuell im Bereich der Hauptabstrahlrichtung angeordnet ist. Auch diesbezüglich kann der Rotationsparameter genutzt werden. Bezüglich der zuvor beschriebenen Filterung ist das Vorliegen der Hauptabstrahlrichtung dahingehend vorteilhaft, da aufgrund dieser Richtcharakteristik ein weiterer Filtereffekt quasi automatisch realisiert wird, ohne dass hierfür konkrete Auswertungsschritte durchgeführt werden müssen. So ist bei typischerweise vorhandenen Richtcharakteristiken die Zahl der Funksender, deren Funksignale gleichzeitig mittels einem der Funkempfänger empfangen werden, begrenzt, wodurch die oben beschriebene Filterung deutlich vereinfacht wird.

Die Funksender können jeweils wenigstens eine Patch-Antenne umfassen oder sein, mittels der die Funksingale generierbar sind. Die Patch-Antenne weist typischerweise eine Richtcharakteristik und mithin eine Hauptabstrahlrichtung auf. Die Antenne, insbesondere die Patch-Antenne, kann auf einer Leiterplatte des Funksenders integriert sein. Die Patch-Antenne kann etwa als eine, insbesondere rechteckige, Metallfläche, die auf der Leiterplatte angeordnet ist, realisiert sein.

Denkbar ist, dass wenigstens einer der Funkempfänger eine Hauptempfangsrichtung aufweist, die eine bezüglich des Empfangs der Funksignale sensitive Raumrichtung betrifft. Das bedeutet, dass mittels des Funkempfängers nur Funksignale empfangen werden können, die aus der Richtung der Hauptempfangsrichtung auf den Funkempfänger auftreffen.

Bevorzugt weist die Hauptabstrahlrichtung bei wenigstens einem der Funksender und die Hauptempfangsrichtung bei wenigstens einem der Funkempfänger entlang und/oder entgegen einer Rotationsrichtung betreffend die Rotation des rotierenden Abschnitts. Die Hauptabstrahlrichtung kann entlang einer Tangentialrichtung des im Querschnitt kreisförmigen, rotierenden Abschnitts verlaufen oder maximal um einen kleinen Winkel, zum Beispiel maximal um 20°, hiervon abweichen. Die Hauptempfangsrichtung kann entlang einer Tangentialrichtung eines im Querschnitt kreisförmigen Aufnahmeabschnitts des stationären Abschnitts verlaufen oder maximal um einen kleinen Winkel, zum Beispiel maximal um 20°, hiervon abweichen.

Bevorzugt sind die Funksender jeweils einer vorwärts gerichteten Funksendermenge oder einer rückwärts gerichteten Funksendermenge zugeordnet. Für die Funksender der vorwärts gerichteten Funksendermenge ist vorgesehen, dass deren Hauptabstrahlrichtungen entlang der Rotationsrichtung weisen. Für die Funksender der rückwärts gerichteten Funksendermenge ist vorgesehen, dass deren Hauptabstrahlrichtungen entgegen der Rotationsrichtung weisen. Besonders bevorzugt sind auch die Funkempfänger jeweils einer vorwärts gerichteten Funkempfängermenge oder einer rückwärts gerichteten Funkempfängermenge zugeordnet. Für die Funkempfänger der vorwärts gerichteten Funkempfängermenge ist vorgesehen, dass deren Hauptempfangsrichtungen entlang der Rotationsrichtung weisen. Für die Funkempfänger der rückwärts gerichteten Funkempfängermenge ist vorgesehen, dass deren Hauptempfangsrichtungen entgegen der Rotationsrichtung weisen. Die Übertragungspaare werden derart gebildet, dass diese jeweils entweder einen der Funksender der vorwärts gerichteten Funksendermenge und einen der Funkempfänger der rückwärts gerichteten Funkempfängermenge oder einen der Funksender der rückwärts gerichteten Funksendermenge und einen der Funkempfänger der vorwärts gerichteten Funkempfängermenge umfassen. In den anderen Fällen ist die Bildung des Übertragungskanals nicht möglich, da die Hauptabstrahlrichtung und die Hauptempfangsrichtung im Wesentlichen in dieselbe Richtung weisen, was die Übertragung der Funksignale ausschließt. Dieser zusätzliche Selektionseffekt bewirkt eine weitere Erhöhung, nämlich eine Verdopplung, der maximal möglichen Datenübertragungsrate, ohne dass hierdurch die oben beschriebene Filterung komplexer wird.

Gemäß der dritten erfindungsgemäßen Ausführung wird das der vorliegenden Erfindung zugrunde liegende Problem bei einem Verfahren der eingangs genannten Art dadurch gelöst, dass von den Funksendern zu den Funkempfängern übertragene Funksignale in wenigstens einen zu den Funkempfängern führenden und aus einem dielektrischen Material bestehenden Signalleiter eingekoppelt werden, wobei der Signalleiter eine sich quer zu einer Erstreckungsrichtung ändernde Permittivität aufweist.

Die Erfindung beruht hinsichtlich der dritten erfindungsgemäßen Ausführung auf dem Gedanken, dass aufgrund der sich ändernden Permittivität eine Ausbreitungsrichtung der Funksignale in dem Signalleiter zu der Erstreckungsrichtung des Signalleiters hin umgelenkt wird, bis diese beiden Richtungen parallel zueinander verlaufen. Die Funksignale können seitlich in den Signalleiter eingekoppelt werden. Hierbei kann der Winkel zwischen dem Signalleiter respektive dessen Erstreckungsrichtung und der Ausbreitungsrichtung der Funksignale größer als 0° und kleiner als 90°, bevorzugt kleiner als 45°, sein.

Sofern sich diese Permittivität nicht ändern würde, dann würden die Funksignale entlang der Querrichtung durch den Signalleiter verlaufen und an der der Einkopplungsseite gegenüberliegenden Seite wieder aus dem Signalleiter austreten. Stattdessen bewirkt die Änderung der Permittivität, insbesondere ein Anstieg der Permittivität, die bereits angesprochene Angleichung der Ausbreitungsrichtung der Funksignale an die Erstreckungsrichtung, sodass die Funksignale entlang der Erstreckungsrichtung innerhalb des Signalleiters geführt werden. Anders ausgedrückt führt die sich quer oder senkrecht zu der Erstreckungsrichtung ändernde Permittivität quasi zu einer stetigen Brechung der elektromagnetischen Welle. Eine zunächst gewinkelt zu der Erstreckungsrichtung verlaufende Ausbreitungsrichtung der Funksignale, die unmittelbar nach dem Einkoppeln in den Signalleiter vorliegt, wird aufgrund der sich ändernden Permittivität in die Richtung der Erstreckungsrichtung umgelenkt, und zwar bis die Ausbreitungsrichtung der Erstreckungsrichtung entspricht.

Mithin verläuft ein Teil des Übertragungsweges, den die Funksignale bei der Übertragung von dem Funksender zum Funkempfänger zurücklegen, durch den Signalleiter, wodurch eine gezielte Führung der Funksignale zu dem Funkempfänger ermöglicht wird. Insbesondere wird hierdurch ein beim Auftreten von Mehrfachreflexionen häufig relevantes Problem, dass Interferenzen der Funksignale zu einer Beeinträchtigung der Datenübertragung führen, gelöst.

Die Permittivität, die oft auch als dielektrische Leitfähigkeit, Dielektrizität oder dielektrische Funktion bezeichnet und häufig mit dem Symbol ε abgekürzt wird, gibt ein Maß für die Polarisationsfähigkeit des Materials an. Bevorzugt ändert sich die Permittivität quer, insbesondere senkrecht, zu der Erstreckungsrichtung kontinuierlich, also ohne dass der Verlauf der Permittivität entlang dieser Querrichtung Sprünge aufweist. Der Bereich der Werte für die Permittivität wird derart gewählt, dass bei dem gewählten Frequenzbereich für die Funksignale eine möglichst schwache Dämpfung eintritt. Der Signalleiter kann aus einem Kunststoff bestehen. Diesbezüglich ist Polystyrol (PS) und/oder Polyethylen (PE) und/oder Polypropylen (PP) und/oder Polytetrafluoräthylen (PTFE) denkbar.

Der Signalleiter kann im Querschnitt, also senkrecht zu der Erstreckungsrichtung, betrachtet eine rechteckige Form aufweisen. Die Erstreckungsrichtung kann entlang der zentralen Faser des Signalleiters verlaufen, also die Mittelpunkte der Querschnittsebenen umfassen. Die Erstreckungsrichtung kann auch als eine Längsrichtung des Signalleiters bezeichnet werden. Der Signalleiter, also dessen Erstreckungsrichtung, erstreckt sich bevorzugt entlang des Umfangs des Aufnahmeabschnitts des stationären Abschnitts. Der Signalleiter respektive die Erstreckungsrichtung bildet mithin einen geschlossenen, bevorzugt kreisförmigen, Ring. Dieser Ring kann die Systemachse konzentrisch umschließen. Die Funksignale werden beim Auftreffen auf den Signalleiter seitlich und radial innenseitig in diesen eingekoppelt. Die Permittivität nimmt radial nach außen zu. Bezüglich der Radialrichtung ist der Gradient der Permittivität mithin positiv, insbesondere konstant.

Die Funkempfänger können innerhalb des Signalleiters angeordnet sein. Anders ausgedrückt können die Funkempfänger in das dielektrische Material eingebettet sein. Die Funksignale treffen also nach dem Einkoppeln in den Signalleiter auf den jeweiligen Funkempfänger auf, ohne dass zuvor eine Auskopplung der Funksignale aus dem Signalleiter erfolgen muss.

Der Signalleiter kann eine sich ändernde geometrische Struktur aufweisen, wobei die sich ändernde Permittivität mittels der sich ändernden geometrischen Struktur realisiert ist. So kann die geometrische Struktur ein Muster bilden, bei dem sich eine Einheits- oder Elementarzelle zyklisch wiederholt. Denkbar ist, dass die Struktur wabenförmig ist, also eine hexagonale Gitterstruktur bildet. Die Größe der Einheits- oder Elementarzelle kann sich zur Realisierung der sich ändernden Permittivität entlang der Querrichtung des Signalleiters ändern. Zur Realisierung der sich ändernden geometrischen Struktur ist denkbar, dass der Signalleiter mittels eines 3D-Druckers hergestellt ist.

Vor allem bezüglich der dritten Ausführung gilt, dass die im Rahmen der zweiten Ausführung erläuterten Aspekte gleichermaßen hierauf anwendbar sind. Dies gilt im Besonderen für das oben hinsichtlich der sich entlang respektive entgegen der Rotationsrichtung weisenden Hauptabstrahlrichtungen und Hauptempfangsrichtungen Erläuterte. So können die Funksignale entlang zweier entgegengesetzter Ausbreitungsrichtungen durch den ringförmigen Signalleiter geführt werden.

Bezüglich aller drei Ausführungen ist denkbar, dass der stationäre Abschnitt den oder einen zylindrischen Aufnahmebereich aufweist, in dem der rotierende Abschnitt angeordnet ist. Die die Funksender können entlang eines Umfangs des rotierenden Abschnitts angeordnet sein. Die Funkempfänger können entlang eines Umfangs des Aufnahmebereichs angeordnet sein. Bevorzugt ist der Aufnahmeabschnitt ein hohler, insbesondere kreiszylindrischer, Freiraum, der von einem Gehäuse des stationären Abschnitts begrenzt wird. Der rotierende Abschnitt ist entsprechend ebenfalls bevorzugt zylindrisch, insbesondere kreiszylindrisch, ausgebildet und gegebenenfalls über ein Ringlager mit dem stationären Abschnitt verbunden. Die Rotation des rotierenden Abschnitts kann mittels eines Antriebsmittel, etwa eines Elektromotors, der mittels der Steuerungseinrichtung angesteuert wird, bewerkstelligt werden. Typischerweise kann die Frequenz der Rotation bis zu mehreren hundert Umdrehungen pro Minute betragen.

Die Funksender und/oder die Funkempfänger können äquidistant entlang des Umfangs angeordnet sein. Insgesamt können beispielsweise bis zu 50 Funksender und bis zu 50 Funkempfänger vorgesehen sein. Die Funksender können entlang eines Außenumfangs des rotierenden Abschnitts angeordnet sein. Die Funkempfänger können entlang eines Innenumfangs des Aufnahmeabschnitts angeordnet sein. Die Funksender und die Funkempfänger sind bevorzugt entlang der Linien zweier konzentrische Kreise, die den jeweiligen Umfang beschreiben, angeordnet.

Neben dem erfindungsgemäßen Verfahren betrifft die vorliegende Erfindung überdies eine medizinische Bildgebungseinrichtung. Diese umfasst ein medizinisches Bildgebungsgerät, insbesondere ein Computertomographiegerät, mit einem rotierenden Abschnitt und einem stationären Abschnitt, wobei an dem rotierenden Abschnitt mehrere Funksender und an dem stationären Abschnitt mehrere Funkempfänger angeordnet sind, wobei mittels der Funksender und der Funkempfänger eine Datenübertragung von dem rotierenden Abschnitt zu dem stationären Abschnitt durchführbar ist.

Gemäß der ersten erfindungsgemäßen Ausführung wird das der vorliegenden Erfindung zugrunde liegende Problem bei einer medizinische Bildgebungseinrichtung gemäß dem zuvor Beschriebenen dadurch gelöst, dass eine Steuerungseinrichtung vorgesehen ist, die dazu eingerichtet ist, Steuersignale zur Steuerung des Betriebs der Funksender und der Funkempfänger derart zu generieren, dass wenigstens ein Übertragungspaar gebildet wird, das einerseits den Funksender oder einen der Funksender und andererseits den Funkempfänger oder einen der Funkempfänger umfasst, wobei bei dem Übertragungspaar oder wenigstens einem der Übertragungspaare ein drahtloser Übertragungskanal zur Übertragung von Funksignalen von dem Funksender zu dem Funkempfänger gebildet wird und eine die Funksignale betreffende Hauptabstrahlrichtung des Funksenders zu der Richtung des Funkempfängers nachgeführt wird.

Gemäß der zweiten erfindungsgemäßen Ausführung wird das der vorliegenden Erfindung zugrunde liegende Problem bei einer medizinische Bildgebungseinrichtung gemäß dem oben Beschriebenen dadurch gelöst, eine Steuerungseinrichtung vorgesehen ist, die dazu eingerichtet ist, Steuersignale zur Steuerung des Betriebs der Funksender und der Funkempfänger derart zu generieren, dass mehrere separate Übertragungspaare, die jeweils einen der Funksender und einen der Funkempfänger umfassen, gebildet werden, wobei bei den Übertragungspaaren jeweils Funksignale von dem Funksender zu dem Funkempfänger übertragen werden, wobei zu den mittels der Funkempfänger empfangenen Funksignalen jeweils wenigstens eine Zuordnungsinformation ermittelt wird, die denjenigen Funksender beschreibt, der dieses Funksignal generiert hat, wobei bei den Übertragungspaaren jeweils mittels der wenigstens einen Zuordnungsinformation diejenigen von dem Funkempfänger empfangenen Funkdaten ausgefiltert werden, die nicht von dem Funksender dieses Übertragungspaars generiert wurden.

Gemäß der dritten erfindungsgemäßen Ausführung wird das der vorliegenden Erfindung zugrunde liegende Problem bei einer medizinische Bildgebungseinrichtung gemäß dem oben Beschriebenen dadurch gelöst, dass von den Funksendern zu den Funkempfängern übertragene Funksignale in wenigstens einen zu den Funkempfängern führenden und aus einem dielektrischen Material bestehenden Signalleiter einkoppelbar sind, wobei der Signalleiter eine sich quer zu einer Erstreckungsrichtung ändernde Permittivität aufweist.

Auch bezüglich der erfindungsgemäßen medizinischen Bildgebungseinrichtung gilt, dass die für eine der Ausführungen erläuterten Vorteile, Merkmale und Aspekte auch auf die anderen Ausführungen anwendbar sind, sofern die jeweiligen Ausführungen diesbezüglich nicht unvereinbar voneinander abweichen. Überdies gilt, dass alle im Zusammenhang mit dem erfindungsgemäßen Verfahren erläuterten Vorteile, Merkmale und Aspekte gleichermaßen auf die erfindungsgemäße medizinische Bildgebungseinrichtung übertragbar sind und umgekehrt.

Weitere Vorteile, Einzelheiten und Merkmale der vorliegenden Erfindung ergeben sich aus den nachfolgend dargelegten Ausführungsbeispielen sowie anhand der Figuren. Diese zeigen schematisch:
- Fig. 1: eine perspektivische Ansicht auf eine erfindungsgemäße medizinische Bildgebungseinrichtung gemäß einem Ausführungsbeispiel,
- Fig. 2: eine schematische Ansicht auf ein medizinisches Bildgebungsgerät der medizinischen Bildgebungseinrichtung der Fig. 1 mit Blickrichtung entlang einer Systemachse, wobei die erfindungsgemäße medizinische Bildgebungseinrichtung sowie das erfindungsgemäße Verfahren jeweils gemäß einer ersten Ausführung anhand der Fig. 2 erläutert wird,
- Fig. 3: dieselbe Ansicht wie Fig. 2, wobei anhand dieser Figur die erfindungsgemäße medizinische Bildgebungseinrichtung sowie das erfindungsgemäße Verfahren jeweils gemäß einer ersten Alternative einer zweiten Ausführung erläutert wird,
- Fig. 4: dieselbe Ansicht wie Fig. 2, wobei anhand dieser Figur die erfindungsgemäße medizinische Bildgebungseinrichtung sowie das erfindungsgemäße Verfahren jeweils gemäß einer zweiten Alternative der zweiten Ausführung erläutert wird,
- Fig. 5: dieselbe Ansicht wie Fig. 2, wobei anhand dieser Figur die erfindungsgemäße medizinische Bildgebungseinrichtung sowie das erfindungsgemäße Verfahren jeweils gemäß einer dritten Ausführung erläutert wird, und
- Fig. 6: einen Längsschnitt durch einen Signalleiter des medizinischen Bildgebungsgeräts der Fig. 5.

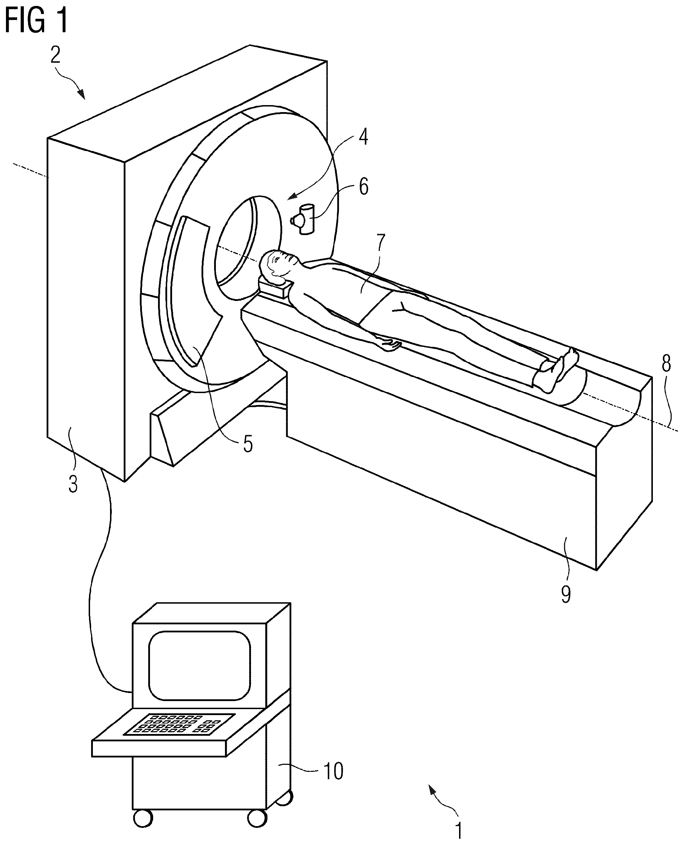

Fig. 1 zeigt eine erfindungsgemäße medizinische Bildgebungseinrichtung 1 mit einem medizinischen Bildgebungsgerät 2, dass vorliegend ein Computertomographiegerät ist. Das Bildgebungsgerät 2 umfasst einen stationären Abschnitt 3 und einen rotierenden Abschnitt 4, wobei der rotierende Abschnitt 4 den Gantry des Computertomographiegeräts bildet. Seitens des rotierenden Abschnitts 4 ist ein Strahler-Detektor-System bestehend aus einem modular aufgebauten Röntgendetektor 5 und der gegenüberliegenden Röntgenquelle 6 vorgesehen. Alternativ können an dem rotierenden Abschnitt 4 auch mehrere Strahler-Detektor-Systeme vorgesehen sein.

Ein Gehäuse des stationären Abschnitts 3 begrenzt einen kreiszylindrischen Aufnahmeabschnitt, in dem der rotierende Abschnitt 4 angeordnet ist und in dem sich im Betrieb ein Röntgen-Messfeld des aktiven Strahler-Detektor-Systems ausbildet. Zur Messung wird ein Patient 7, der sich auf einer in Richtung einer Systemachse 8 verfahrbaren Patientenliege 9 befindet, kontinuierlich oder schrittweise durch das Messfeld geschoben, während der rotierende Abschnitt 4 um die Systemachse 8 rotiert. Hierbei wird die Schwächung der von der Röntgenröhre ausgesandten Röntgenstrahlung durch den Patienten 7 pixelweise mittels Sensoren gemessen, wobei bevorzugt direktkonvertierende Sensormaterialien, etwa ein Szintillator, verwendet werden und die einfallenden Röntgen-Photonen energieaufgelöst gezählt werden.

Die so ermittelten Detektordaten werden im Rahmen einer drahtlosen Datenübertragung von dem rotierenden Abschnitt 4 zu dem stationären Abschnitt 3 übertragen. Die diesbezügliche Steuerung sowie auch die Auswertung der empfangenen Messdaten zur Realisierung der medizinischen Bildgebung erfolgt mittels einer Steuerungseinrichtung 10 der medizinische Bildgebungseinrichtung 1, und zwar mit Hilfe einer dort implementierten und im Betrieb ausgeführten Software. Die Steuerungseinrichtung 10 ist zudem dazu eingerichtet, den Betrieb von Funksendern 11, 13, 17, 18 und Funkempfängern 12, 15, 19, 20 zu steuern. Obgleich nachfolgend eine unidirektionale Datenübertragung von dem rotierenden Abschnitt 4 zu dem stationären Abschnitt 3 erläutert wird, ist grundsätzlich auch denkbar, dass eine Datenübertragung in die entgegengesetzte Richtung erfolgt und eine bidirektionale Kommunikation stattfindet, etwa zu Steuerungszwecken betreffend die Detektoren. Eine Übertragung von Daten von dem stationären Abschnitt 3 zu dem rotierenden Abschnitt 4 ist zudem oder alternativ auch mittels eines Schleifringsystems denkbar, bei dem ein elektrischer Schleifkontakt eine die Datenübertragung ermöglichende Kopplung zwischen dem rotierenden Abschnitt 4 und dem stationären Abschnitt 3 herstellt.

Bezüglich der Rotation des rotierenden Abschnitts 4 ist die Steuerungseinrichtung 10 dazu eingerichtet, einen in den Figuren nicht gezeigten und mit dem rotierenden Abschnitt 4 gekoppelten Elektromotor anzusteuern. Der Elektromotor ist mit dem rotierenden Abschnitt 4 derart gekoppelt, dass mittels diesem die Rotation des rotierenden Abschnitts 4 gemäß vorgegebener Rotationsparameter realisierbar ist. Die Rotationsparameter, die die Rotation des rotierenden Abschnitts 4 beschreiben, werden etwa anhand einer Nutzereingabe und/oder eines fest vorgegebenen Programmablaufs, der seitens der Steuerungseinrichtung 10 hinterlegt ist, vorgegeben. Einer der Rotationsparameter betrifft die Rotationsfrequenz des rotierenden Abschnitts 4. Ein weiterer Rotationsparameter betrifft eine Phase dieser Rotation, also die bei einem Nulldurchgang der Rotation vorliegende Stellung des rotierenden Abschnitts 4.

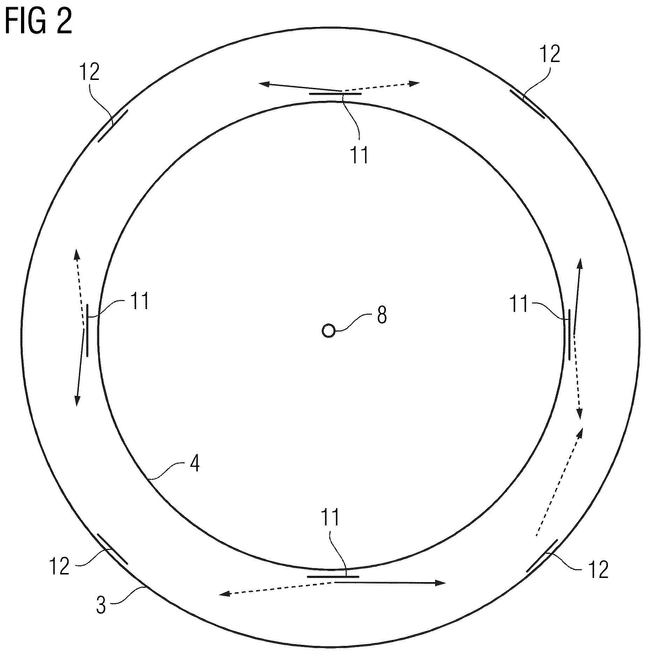

Nachfolgend wird anhand der Fig. 2 eine erste Variante der Erfindung erläutert, und zwar betreffend die medizinische Bildgebungseinrichtung 1 sowie das erfindungsgemäße Verfahren. Fig. 2 zeigt eine Ansicht auf das Bildgebungsgerät 2 mit Blickrichtung entlang der Systemachse 8. Die Abschnitte 3, 4 sind, gleichermaßen die die anderen in dieser Figur gezeigten Komponenten, nur äußerst schematisch angedeutet.

Zur Realisierung der Datenübertragung sind an dem rotierenden Abschnitt 4 angeordnete Funksender 11 und an dem stationären Abschnitt 3 angeordnete Funkempfänger 12 vorgesehen, nämlich beispielhaft insgesamt jeweils vier. Die Funksender 11 sind äquidistant entlang eines Außenumfangs des zylinderförmigen rotierenden Abschnitts 4 angeordnet. Die Funkempfänger 12 sind äquidistant entlang eines Innenumfangs des zylinderförmigen Aufnahmeabschnitts angeordnet. Die Funksender 11 und die Funkempfänger 12 sind jeweils entlang einer gedachten Kreislinie zweier konzentrische Kreise angeordnet, durch deren Mittelpunkt die Systemachse 8 verläuft.

Bezogen auf die Fig. 2 sei angenommen, dass der rotierende Abschnitt 4 entlang einer Rotationsrichtung rotiert, vorliegend beispielhaft im Uhrzeigersinn. Zur Definition von Raumrichtungen, die sich in Fig. 2 radial von der Systemachse 8 weg erstrecken, sei angenommen, dass die Richtung senkrecht nach oben, die einer Zwölf-Uhr-Stellung entspricht, einem Wert von 0° sowie 360° zugeordnet wird. Ausgehend von der 0°-Stellung betrifft eine Winkelangabe bis 360° die Rotation entlang des Uhrzeigersinns. So entspricht eine Winkelangabe von 90° einer Drei-Uhr-, eine Winkelangabe von 180° einer Sechs-Uhr- und eine Winkelangabe von 270° einer Neun-Uhr-Stellung.

Die Funksender 11 befinden sich in Fig. 2 bei 0°, 90°, 180° und 270°, wobei diese Werte während der Rotation des rotierenden Abschnitt 4 entsprechend zunehmen. Anhand der bekannten Rotationsparameter werden die Positionen der Funksender 11 in Abhängigkeit der Zeit bestimmt. Die Positionen der Funkempfänger 12 sind ortsfest und werden als bekannt vorausgesetzt. Die Funkempfänger 12 befinden sich in der Fig. 2 konstant bei 45°, 135°, 225° und 315°. Hieraus werden, ebenfalls zeitabhängig, die Relativpositionen zwischen den Funksendern 11 und den Funkempfängern 12 bestimmt.

In Abhängigkeit der Relativpositionen zwischen den Funksendern 11 und den Funkempfängern 12 werden vier Übertragungspaare gebildet, die jeweils einen der Funksender 11 und einen der Funkempfänger 12 umfassen. Hierzu wird jedem der Funksender 11 genau derjenige Funkempfänger 12 zugeordnet, der momentan bezüglich aller Funkempfänger 12 den kürzesten Abstand zu diesem Funksender 11 aufweist. Mittels der Übertragungspaare wird jeweils ein drahtloser Übertragungskanal zur Übertragung von Funksignalen von dem jeweiligen Funksender 11 zu dem jeweiligen Funkempfänger 12 gebildet. Die Datenübertragung erfolgt über diese Übertragungskanäle.

Da sich die Relativabstände zwischen den Komponenten der Übertragungspaare laufend ändern, ist es zur Aufrechterhaltung der Datenübertragung während der Rotation des rotierenden Abschnitts 4 erforderlich, dass die Übertragungspaare laufend aktualisiert werden. Zur Bestimmung der Aktualisierungszeitpunkte, zu denen eine Neuzuordnung der Übertragungspaare erforderlich ist und vorgenommen wird, werden die Rotationsparameter genutzt. Unter Bezugnahme auf den bei 0° angeordneten Funksender 11 in Fig. 2 wird deutlich, dass sich dieser für den gezeigten Zeitpunkt zwischen zwei Funkempfängern 12 befindet, nämlich zwischen dem Funkempfänger 12 rechts oben bei 45° und dem Funkempfänger 12 links oben bei 315°. Unmittelbar vor dem in Fig. 2 gezeigten Zeitpunkt ist derjenige Funkempfänger 12, der zu diesem Funksender 11 den kürzesten Abstand aufweist, der Funkempfänger 12 bei 315°. Die entsprechende Übertragung von Funksignalen ist in Fig. 2 mittels des durchgezogenen Pfeils angedeutet. Unmittelbar nach dem in Fig. 2 gezeigten Zeitpunkt ist derjenige Funkempfänger 12, der zu diesem Funksender 11 den kürzesten Abstand aufweist, der Funkempfänger 12 bei 45°. Die entsprechende Übertragung von Funksignalen ist in Fig. 2 mittels des gestrichelten Pfeils angedeutet. Das den Funksender 11 bei 0° aufweisende Übertragungspaar wird mithin zu dem in der Fig. 2 gezeigten Zeitpunkt, der folglich einer der Aktualisierungszeitpunkte ist, aktualisiert. Dies gilt analog für die drei weiteren Übertragungspaare.

Neben den Rotationsparametern wird ein Qualitätsparameter bestimmt, der eine Intensität und mithin Qualtiät der mittels des jeweiligen Funkempfängers 12 empfangenen Funksignale betrifft. So hängt die Intensität respektive Signalstärke der Funksignale von dem Abstand zwischen dem Funksender 11 und dem Funkempfänger 12 sowie von dem Umstand ab, ob aktuell eine direkte Sichtlinie zwischen dem Funksender 11 und dem Funkempfänger 12 gegeben ist. Unmittelbar vor dem in Fig. 2 gezeigten Zeitpunkt ist eine direkte Sichtlinie bei dem Übertragungspaar umfassend den Funksender 11 bei 0° und den Funkempfänger 12 bei 315° vorhanden. Mit Erreichen der der in Fig. 2 gezeigten Stellung wird diese Sichtlinie durch den rotierenden Abschnitt 4 unterbrochen, wobei eine direkte Sichtlinie bei dem Übertragungspaar umfassend den Funksender 11 bei 0° und den Funkempfänger 12 bei 54° entsteht. Dies schlägt sich entsprechend in dem Qualitätsparameter nieder, bei dem bezüglich des Funksenders 11 bei 0° und des Funkempfängers 12 bei 315° ein schlagartiger Abfall der Intensität und bezüglich des Funksenders 11 bei 0° und des Funkempfängers 12 bei 45° ein schlagartiger Anstieg der Intensität eintritt. Obgleich der Qualitätsparameter erfindungsgemäß anstelle der Rotationsparameter zur Bestimmung der Aktualisierungszeitpunkte genutzt werden kann, werden vorliegend alle Parameter für diesen Zweck genutzt. So wird der Qualitätsparameter vorliegend zur Verifizierung der anhand der Rotationsparameter bestimmten Aktualisierungszeitpunkte verwendet. Zudem wird der Rotationsparameter betreffend die Phase anhand des Qualitätsparameter aktualisiert respektive berichtigt, etwa um eine sich über die Zeit immer weiter vergrößernde Abweichung der Phase zu vermeiden.

Ein weiterer Aspekt des in Fig. 2 gezeigten Ausführungsbeispiels betrifft eine Hauptabstrahlrichtung der Funksender 11. Die Hauptabstrahlrichtung bezeichnet diejenige von dem jeweiligen Funksender 11 ausgehende Raumrichtung, entlang der die Intensität der mittels dieses Funksenders 11 generierten und die Funksignale bildenden elektromagnetischen Wellen ein Maximum aufweist. So ist diesbezüglich vorgesehen, dass die Hauptabstrahlrichtung in die Richtung des Funkempfängers 12 des jeweiligen Übertragungspaars nachgeführt wird. Überdies wird die Hauptabstrahlrichtung zum Aktualisierungszeitpunkt derart geändert, dass diese von der Position des Funkempfängers 12 des Übertragungspaars vor der Aktualisierung zu der Position des Funkempfängers 12 des Übertragungspaars nach der Aktualisierung verschwenkt wird.

Die veränderbare Hauptabstrahlrichtung wird dadurch realisiert, dass die Funksender 11 jeweils eine elektronisch schwenkbare Antenne umfassen, nämlich eine Beamforming-Antenne, die eine Phased-Array-Antenne mit zwischen 4 und 64 Antennenkomponenten ist. Mittels einer elektronischen Ansteuerung dieser Antennenkomponenten seitens der Steuerungseinrichtung 10 ist die Hauptabstrahlrichtung veränder- und einstellbar. Die Steuerung der Nachführung der Hauptabstrahlrichtung erfolgt vorliegend anhand der Rotationsparameter sowie des Qualitätsparameters.

Bei dieser Ausführungsform wird eine Multi-Gigabit-Übertragung realisiert, wobei als Frequenzbereich exemplarisch das ISM-Band bei 60 GHz benutzt wird. Verwendet werden vier Übertragungskanäle mit einer Übertragungsbandbreite von je 2 GHz, wobei sich insgesamt eine Datenrate von etwa 25 Gb/s ergibt. Die konkrete Anzahl der Funksender 11 und Funkempfänger 12 ist nur beispielhaft zu verstehen, sodass, etwa wenn eine Datenrate von 200 Gb/s erforderlich ist, auch acht getrennte Übertragungskanäle und mithin jeweils acht Funksender 11 und Funkempfänger 12 vorgesehen sein können.

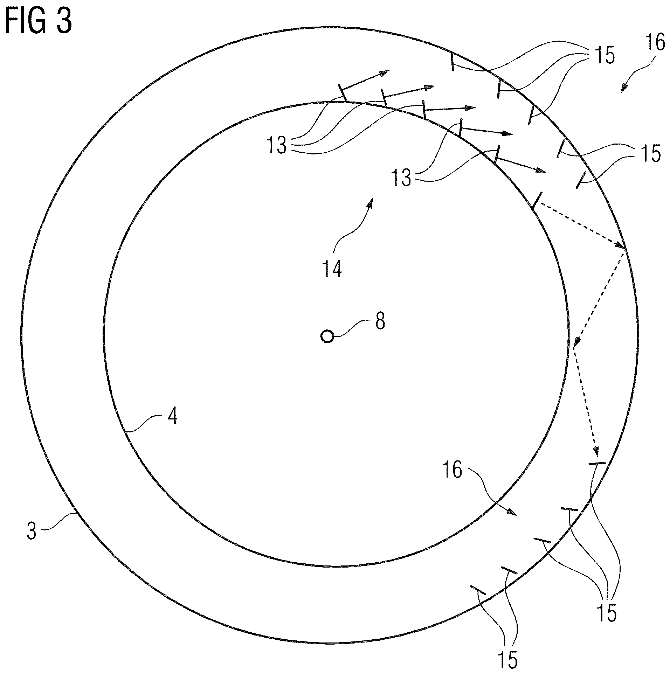

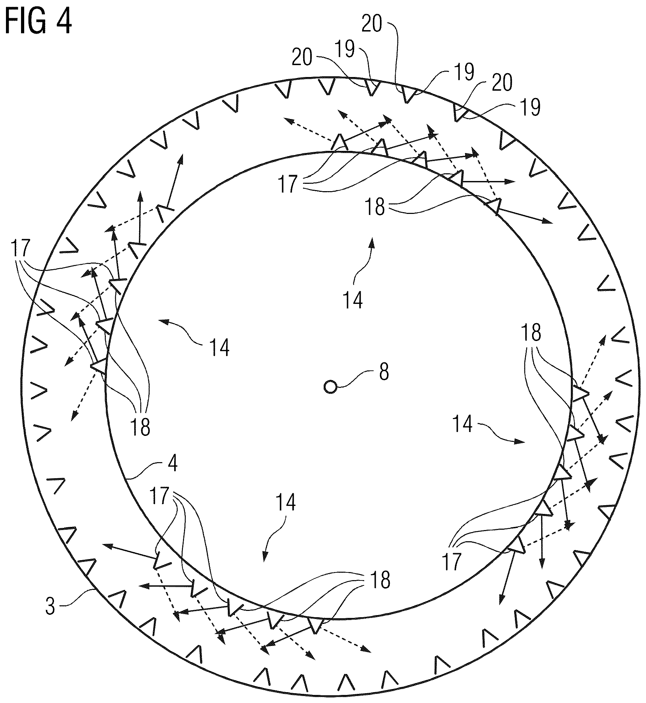

Nachfolgend wird anhand der Figuren 3 und 4 eine zweite Variante der vorliegenden Erfindung erläutert. Fig. 3 betrifft hierbei eine erste und Fig. 4 eine zweite Alternative der zweiten Variante. Zunächst wird auf Fig. 3 Bezug genommen, die dieselbe Ansicht auf das Bildgebungsgerät 2 zeigt wie Fig. 2. Bei dem in der Fig. 3 gezeigten Ausführungsbeispiel wird bei der erfindungsgemäßen medizinischen Bildgebungseinrichtung 1 ein sogenanntes MIMO-Konzept realisiert.

So sind die am dem rotierenden Abschnitt 4 angeordneten Funksender 13 vier Funksendergruppen 14 zugeordnet, die jeweils mehrere gruppiert entlang des Umfangs des rotierenden Abschnitts 4 angeordnete Funksender 13 umfassen. Die die Funksender 13 der jeweiligen Funksendergruppe 14 sind jeweils unmittelbar benachbart zueinander angeordnet, wobei zu den Funksendern 13 der anderen Funksendergruppen 14 ein diesbezüglich größerer Abstand gegeben ist. Folglich sind die Funksender 13 jeder der Funksendergruppen 14 gehäuft nebeneinander und beabstandet zu den Funksendern 13 der anderen Funksendergruppen 14 angeordnet. Der Übersichtlichkeit halber ist in Fig. 3 nur eine der Funksendergruppen 14 gezeigt, nämlich die im Bereich zwischen 0° und 60° vorgesehene. Die anderen Funksendergruppen sind in den Bereichen zwischen 90° und 150°, zwischen 180° und 240° und zwischen 270° und 330° angeordnet. Analog sind auch die Funkempfänger 15 jeweils einer von vier Funkempfängergruppen 16 zugeordnet, wobei in Fig. 3 zwei der Funkempfängergruppen 16 dargestellt sind.

Im Unterschied zu dem anhand der Fig. 2 erläuterten Ausführungsbeispiel ist bei dem in Fig. 3 gezeigten Ausführungsbeispiel vorgesehen, dass die Funksender 13 eine bezüglich des rotierenden Abschnitts 4 feste Hauptabstrahlrichtung aufweisen. So umfassen die Funksender 13 hierzu jeweils eine Patch-Antenne, mittels der die Funksignale gerichtet abgestrahlt werden. Die Hauptabstrahlrichtung sind in Fig. 3 anhand der Pfeile angedeutet, wobei erkennbar ist, dass eine Übertragung der Funksignale von den Funksendern 13 zu den Funkempfängern 15 auch über Reflexionen an dem stationären Abschnitt 3 und dem rotierenden Abschnitt 4 denkbar sind.

Analog zu der Hauptabstrahlrichtung der Funksender 13 ist auch bei den Funkempfängern 15 vorgesehen, dass diese jeweils eine Hauptempfangsrichtung aufweisen, die eine bezüglich des Empfangs der Funksignale sensitive Raumrichtung betrifft. Aus der Fig. 3 wird ersichtlich, dass bei dem dort gezeigten Ausführungsbeispiel die Hauptabstrahlrichtung entlang der Rotationsrichtung betreffend die Rotation des rotierenden Abschnitts 4 weist. Das heißt, dass die Hauptabstrahlrichtung, die entlang der in Fig. 3 eingezeichneten Pfeile weist, im Wesentlichen entlang der Tangentialrichtung des rotierenden Abschnitts 4 zeigt. Die Hauptempfangsrichtungen der Funkempfänger 15 sind entsprechend entgegengesetzt ausgerichtet.

Bezüglich der Datenübertragung ist vorgesehen, dass mehrere Übertragungspaare gebildet werden, die jeweils einen der Funksender 13 und einen der Funkempfänger 15 umfassen. Das im Zusammenhang mit der ersten Variante zu den Übertragungspaaren Erläuterte gilt, abgesehen von dem nachfolgend Dargelegten, auch für die Übertragungspaare bei der zweiten Variante. Ein Unterschied ist, dass aufgrund der bezüglich des rotierenden Abschnitts 4 festen Hauptabstrahlrichtungen der Funksender 13 und der bezüglich des stationären Abschnitts 3 festen Hauptempfangsrichtungen der Funkempfänger 15 eine Datenübertragung nur in denjenigen Momenten oder Zeitfenstern erfolgen kann, wenn die feste Hauptabstrahlrichtung aktuell die Position des Funkempfängers 15, der die diesbezüglich entgegengesetzte Hauptempfangsrichtung aufweist, überstreicht. Bezogen auf die Fig. 3 ist dies für die Funksender 13 der rechts oben eingezeichneten Funksendergruppe 14 und die Funkempfänger der rechts oben eingezeichneten Funkempfängergruppe 16 der Fall. Im Wesentlichen stimmen die Hauptabstrahlrichtungen und die Hauptempfangsrichtungen richtungsmäßig derart überein, dass die von den Funksendern 13 dieser Funksendergruppe 14 generierten Funksignale von den Funkempfängern 15 dieser Funkempfängergruppe 16 empfangen werden.

Problematisch hierbei ist der Umstand, dass jeder der Funkempfänger 15 der in Fig. 3 rechts oben gezeigten Funkempfängergruppe 16 die Funksignale aller oder zumindest einiger der Funksender 13 der in dieser Figur eingezeichneten Funkempfängergruppe 14 empfängt. Ein weiteres Problem diesbezüglich stellt der Umstand dar, dass zudem die Funksignale weiterer, in Fig. 3 nicht eingezeichneter Funksendergruppen 14 mittels der eingezeichneten Funkempfängergruppe 14 empfangen werden. Dies liegt, obgleich keine unmittelbaren Sichtlinien vorhanden sind, an dem Umstand, dass diese Funksignale über Reflexionen respektive Mehrfachreflexionen zu dieser Funkempfängergruppe 14 gelangen. Ein denkbarer Übertragungsweg einer dieser Funksignale ist bezüglich der in Fig. 3 eingezeichneten Funksendergruppe 14 bezüglich des aktuell am weitesten rechts angeordneten Funksenders 13 eingezeichnet.

Aufgrund des soeben erläuterten Umstandes, dass seitens jedem der Funkempfänger 15 nicht nur die Funksignale des im Rahmen der Zuordnung der Übertragungspaare aktuell zugeordneten Funksenders 13, sondern einer Vielzahl der Funksender 13 erfasst werden, ist eine Filterung der Funksignale diesbezüglich erforderlich. Hierdurch wird sichergestellt, dass für jeden der Funkempfänger 15 nur diejenigen Funksignale weiterverarbeitet werden, die von demjenigen Funksender 13 generiert wurden, der dem entsprechenden Übertragungspaar zugeordnet ist. Bezogen auf die Fig. 3 ist in dem aktuellen Zustand bei den beiden rechts oben eingezeichneten Gruppen 14, 16 vorgesehen, dass der erste Funksender 13 von links und der erste Funkempfänger 15 von links, der zweite Funksender 13 von links und der zweite Funkempfänger 15 von links und so weiter jeweils ein Übertragungspaar bilden. Die konkrete Vorgabe der Übertragungspaare erfolgt anhand der auch im Rahmen dieser Alternative erfassten Rotationsparameter, sodass die Hauptabstrahlrichtung des jeweiligen Funksenders 13 und die Hauptempfangsrichtung des jeweiligen Funkempfängers 15 zumindest im Wesentlichen kollinear verlaufen.

Zur Ermöglichung der Filterung der Funksignale ist vorgesehen, dass zu jedem der übertragenen Funksignale jeweils mehrere Zuordnungsinformationen ermittelt werden, anhand derer derjenige Funksender 13 ermittelbar ist, der dieses Funksignal generiert hat. Dieses Vorgehen erlaubt es, dass für jeden der Funkempfänger 15 diejenigen Funksignale verworfen werden, die nicht von dem Funksender 13 stammen, der dem jeweiligen Übertragungspaar zugeordnet ist.

Als eine der Zuordnungsinformationen ist eine Frequenzinformation vorgesehen, die eine Frequenz der das jeweilige Funksignal bildenden elektromagnetischen Wellen betrifft. So können die Funksignale, die von unterschiedlichen Funksendern 13 generiert werden, unterschiedliche Frequenzen aufweisen. Seitens des Funkempfängers 15 ist die jeweilige Frequenz messtechnisch erfassbar, wobei das entsprechende Ergebnis die Frequenzinformation ist.

Bei dem in Fig. 3 gezeigten Ausführungsbeispiel wird mittels jeder der Funkempfängergruppen 14 ein separates MIMO-System realisiert. Das bedeutet, dass die Funksender 13 einer der Funksendergruppen 14 jeweils Funksignale im gleichen Frequenzbereich generieren, wobei die Funksender 13 unterschiedlicher Funksendergruppen 14 jeweils Funksignale in verschiedenen Frequenzbereichen generieren. Hierdurch wird eine Vorfilterung der Funksignale ermöglicht. So werden Gruppenpaare umfassend eine der Funksendergruppen 14 und eine der Funkempfängergruppen 16 gebildet. Bei dem jeweiligen Gruppenpaar ist vorgesehen, dass die Funksender 13 der jeweiligen Funksendergruppe 14 und die Funkempfänger 15 der jeweiligen Funkempfängergruppe 16 jeweils Übertragungspaare bilden. Vorliegend bilden die beiden rechts oben in Fig. 3 eingezeichneten Gruppen 14, 16 eines der Gruppenpaare.

Seitens der Steuerungseinrichtung 10 sind die Frequenzen der mittels der Funksender einer der Funksendergruppen 14 jeweils generierten Funksignale hinterlegt. Im Rahmen der Vorfilterung ist vorgesehen, dass diese bekannte Frequenz oder dieses bekannte Frequenzband mit der jeweils erfassten Frequenzinformation eines der Funkempfänger 15 der Funkempfängergruppe 16 abgeglichen wird, wobei eine Weiterverarbeitung des jeweiligen Funksignals im Rahmen der Durchführung der Filterung nur dann erfolgt, wenn eine Übereinstimmung vorliegt.

Dies gilt für jedes der vier Gruppenpaare, sodass insgesamt ein vierfaches MIMO-System realisiert wird. Bei diesem ist vorgesehen, dass jeder der Funksender 13 auf einer Bandbreite von 2 GHz und unter Verwendung einer Quadratur-AmplitudenModulation von 16 arbeitet. Die Trennung dieser einzelnen MIMO-Systeme wird durch die zuvor beschriebene Filterung hinsichtlich der Frequenzen ermöglicht. Die Funksender 13 eines der Teilsysteme respektive einer der Funkempfängergruppen 14 arbeiten auf einer gemeinsamen Frequenz, wobei sich die Frequenzen der vier Teilsysteme respektive Funkempfängergruppen 14 untereinander unterscheiden. Denkbar sind diesbezüglich Frequenzen von 60 GHz, 62 GHz, 64 GHz und 66 GHz.

Als eine der Zuordnungsinformationen ist eine Distanzinformation vorgesehen, die eine Distanz zwischen dem jeweiligen Funkempfänger 15 und dem Funksender 13, der dieses Funksignal generiert hat, betrifft. So kann anhand der bekannten Rotationsparameter der Relativabstand zwischen dem Funksender 13 und dem Funkempfänger 15 für jedes der Übertragungspaare berechnet werden, wobei anhand einer erfolgenden Freiraumdämpfung und gegebenenfalls unter Berücksichtigung der Richtcharakteristik eine bei dem Empfang mittels des Funkempfängers 15 des jeweiligen Übertragungspaars zu erwartende Signalstärke für die Funksignale, die von dem Funksender 13 dieses Übertragungspaars generiert wurden, bestimmt werden. Dieses Ergebnis wird mit der Distanzinformationen abgeglichen werden, wobei nur diejenigen Funksignale weiterverarbeitet und nicht verworfen werden, bei denen die Werte übereinstimmen. Die Distanzinformation ist einerseits hinsichtlich der Zuordnung der Funksignale zu den jeweiligen Funksendergruppen 14 respektive der Vorfilterung äußerst zweckdienlich, da, sofern wie vorliegend für die Funksignale eine Frequenz von etwa 60 GHz verwendet wird, eine hohe Freiraumdämpfung gegeben ist, die eine entsprechende Trennschärfe hinsichtlich der Distanzinformation realisiert. Andererseits ist diese Trennschärfe auch groß genug, sodass die Distanzinformation eine Unterscheidung der einzelnen Funksender 13 innerhalb einer der Funksendergruppen 14 und mithin eine finale Filterung ermöglicht.

Gleichwohl ist überdies als eine der Zuordnungsinformation eine Polarisationsinformation vorgesehen, die eine Polarisation des jeweiligen Funksignals betrifft. So weisen die die Funksignale bildenden elektromagnetischen Wellen, die von unterschiedlichen Funksendern 13 generiert werden, unterschiedliche Polarisationen auf, beispielsweise eine horizontale, vertikale, diagonale, zirkuläre oder elliptische Polarisation. Die Antennen der Funksender 13 generieren hierzu entsprechend elektromagnetische Wellen mit unterschiedlichen Polarisationseigenschaften. Seitens des Funkempfängers 15 ist die jeweilige Polarisation messtechnisch erfassbar, wobei das Ergebnis die Polarisationsinformation ist. Zudem ist für jeden Funksender 13 die Polarisation der generierten Funksignale bekannt. Das Ergebnis für die Polarisationsinformation kann hiermit abgeglichen werden, wobei nur diejenigen Funksignale weiterverarbeitet und nicht verworfen werden, bei denen die Polarisationsinformation mit der Polarisation des Funksenders 13 des jeweiligen Übertragungspaars übereinstimmt.

Nachfolgend wird anhand der Fig. 4 eine zweite Alternative der zweiten Variante der Erfindung erläutert. Fig. 4 zeigt dieselbe Ansicht auf das Bildgebungsgerät 2 wie die Figuren 2 und 3. Die bezüglich des anhand der Fig. 3 erläuterten Aspekte gelten, sofern nicht explizit abweichend, gleichermaßen für das in Fig. 4 gezeigte Ausführungsbeispiel. Im Unterschied zur Fig. 3 sind in der Fig. 4 alle vorgesehenen Funksender 17, 18 sowie alle vorgesehenen Funkempfänger 19, 20 eingezeichnet. Während die Funkempfänger 19, 20 gleichmäßig verteilt entlang des Umfangs des stationären Abschnitts 3 angeordnet sind, sind bezüglich der Funksender 17, 18 die bereits zuvor erläuterten vier Funksendergruppen 14 vorgesehen. Den Funkempfängergruppen 16 sind nicht jeweils gehäuft angeordnete Funkempfängern 19, 20 zugeordnet. Stattdessen werden die Funkempfängergruppen 16 anhand der aufgrund der Rotationsparameter bekannten aktuellen Relativstellungen der Funksender 17, 18 relativ zu den Funkempfängern 19, 20 bestimmt. So werden jeweils diejenigen Funkempfänger 19, 20 zu einer Funkempfängergruppen 16 zusammengefasst, die von den Hauptabstrahlrichtungen der Funksender 17, 18 einer der Funksendergruppen 14 erfasst respektive getroffen werden. Diese beiden Gruppen 14, 16 werden zu einem Gruppenpaar zusammengefasst. Jede der Funkempfängergruppen 16 wandert also quasi zusammen mit der Rotation des rotierenden Abschnitts 4 und mithin der zugehörigen Funksendergruppe 4 entlang des Umfangs des stationären Abschnitts 3.

Zusätzlich ist bei dem in Fig. 4 gezeigten Ausführungsbeispiel vorgesehen, dass die Funksender 17 einer vorwärts gerichteten Funksendermenge und die Funksender 18 einer rückwärts gerichteten Funksendermenge zugeordnet sind. Bei den Funksendern 17 weisen die Hauptabstrahlrichtungen entlang und bei den Funksendern 18 entgegen der Rotationsrichtung. Diese Richtungen sind bei den Funksendern 17 mittels durchgezogener und bei den Funksendern 18 mittels gestrichelter Pfeile angedeutet.

Weiterhin sind auch die Funkempfänger 19 einer vorwärts gerichteten Funkempfängermenge und die Funkempfänger 20 einer rückwärts gerichteten Funkempfängermenge zugeordnet. Bei den Funkempfängern 19 weisen die Hauptempfangsrichtungen entlang und bei den Funkempfängern 20 entgegen der Rotationsrichtung. Aufgrund dieser Richtcharakteristiken der Funksender 17, 18 und der Funkempfänger 19, 20 können Übertragungspaare grundsätzlich nur zwischen den Funksendern 17 der vorwärts gerichtete Funksendermenge und Funkempfängern 20 der rückwärts gerichteten Funkempfängermenge und umgekehrt gebildet werden.

Aufgrund dessen, dass bei dem in Fig. 4 gezeigten Ausführungsbeispiel mehrere Funksendergruppen 14 vorgesehen sind, wird analog zu den anhand der Fig. 3 erläuterten Aspekten ein mehrfaches MIMO-System realisiert. Überdies wird aufgrund der diametral entgegengesetzten Richtcharakteristiken beziehungsweise der ferner vorhandenen Aufteilung der Funksender 17, 18 und Funkempfänger 19, 20 in vorwärts und rückwärts gerichtete Gruppen ein Selektionseffekt bezüglich der Empfangbarkeit der Funksignale bei dem Funkempfänger 19, 20 realisiert, sodass das anhand der Fig. 3 beschriebene vierfache MIMO-System zu einem achtfachen MIMO-System verdoppelt wird, ohne dass diesbezüglich ein zusätzlicher Aufwand hinsichtlich der erforderlichen Filterung entsteht.

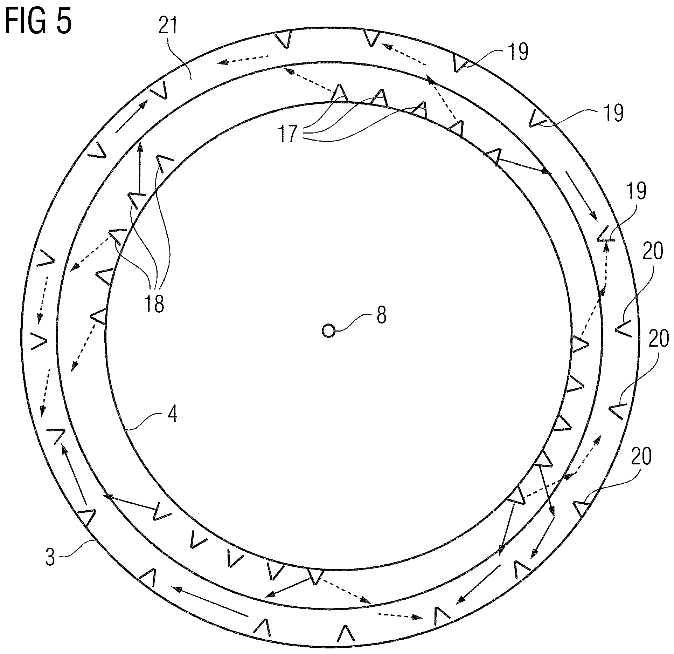

Nachfolgend wird anhand der Fig. 5 eine dritte Variante der vorliegenden Erfindung erläutert. Fig. 5 zeigt dieselbe Ansicht auf das Bildgebungsgerät 2 wie die Figuren 2 bis 4. Abgesehen von den nachfolgend erläuterten Aspekten gilt das im Zusammenhang mit der Fig. 4 Erläuterte gleichermaßen für das anhand der Fig. 5 erläuterte Ausführungsbeispiel. Der Übersichtlichkeit halber sind in Fig. 5 nur einige der die Übertragung der Funksignale andeutenden Pfeile dargestellt.

Im Unterschied zu den zuvor erläuterten Ausführungen ist bei der dritten Ausführung ein Signalleiter 21 vorgesehen, wobei der Übertragungsweg der Funksignale von den Funksendern 17, 18 zu den Funkempfängern 19, 20 zum Teil durch den Signalleiter 21 verläuft. Der Signalleiter 21 ist kreis- und ringförmig und an dem Innenumfang des Aufnahmeabschnitts des stationären Abschnitts 3 befestigt. Die Funksignale treffen seitlich auf die radiale Innenseite des Signalleiters 21 auf, werden in diesen eingekoppelt und propagieren anschließend entlang einer Längs- respektive Erstreckungsrichtung des Signalleiters 21. Die Funkempfänger 19, 20 sind innerhalb des Signalleiters 21 angeordnet, also in dessen Material eingebettet.

Bezogen auf die Fig. 5 verlaufen diejenigen Funksignale, die von den Funksendern 17 der vorwärts gerichteten Funksendermenge generiert werden, im Uhrzeigersinn durch den Signalleiter 21 und werden von einem der Funkempfänger 20 der rückwärts gerichteten Funkempfängermenge empfangen. Diejenigen Funksignale, die von den Funksendern 18 der rückwärts gerichteten Funksendermenge generiert werden, verlaufen gegen den Uhrzeigersinn durch den Signalleiter 21 und werden von einem der Funkempfänger 19 der vorwärts gerichteten Funkempfängermenge empfangen.

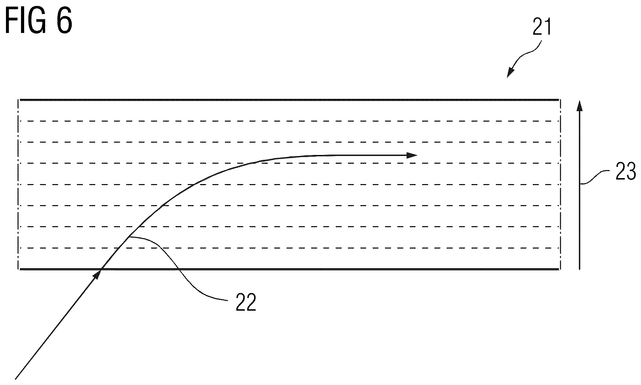

Fig. 6 zeigt eine detaillierte Ansicht eines Abschnitts des Signalleiters 21, wobei der Übertragungsweg eines der Funksignale durch den Pfeil 22 angedeutet ist. Die von der Systemachse 8 radial nach außen weisende Richtung ist mittels des Pfeils 23 angedeutet. Der Signalleiter 21 besteht aus einem dielektrischen Material, nämlich aus einem Kunststoff wie etwa Polystyrol, Polyethylen, Polypropylen und/oder Polytetrafluoräthylen. Hierbei ist vorgesehen, dass dieses Material respektive der Signalleiter 21 eine sich quer zu der Erstreckungsrichtung des Signalleiters 21 ändernde Permittivität aufweist. Die Erstreckungsrichtung steht senkrecht auf der durch den Pfeil 23 angedeuteten Richtung. In Fig. 6 sind gestrichelt Isolinien der Permittivität eingezeichnet.

Die sich senkrecht zu der Erstreckungsrichtung des Signalleiters 21 ändernde Permittivität führt zu einer stetigen Brechung der das jeweilige Funksignal darstellenden elektromagnetischen Welle. Eine zunächst gewinkelt zu der Erstreckungsrichtung verlaufende Ausbreitungsrichtung des Funksignals wird aufgrund der sich ändernden Permittivität zur Erstreckungsrichtung hin umgelenkt, und zwar bis die Ausbreitungsrichtung der Erstreckungsrichtung entspricht. Ab diesem Moment wird das jeweilige Funksignal entlang der Erstreckungsrichtung geführt, und zwar bis dieses den nächsten in dem Signalleiter 21 angeordneten Funkempfänger 19, 20 erreicht.

Die vorliegende Änderung der Permittivität des Signalleiters 21 wird dadurch realisiert, dass dessen Material eine sich ändernde geometrische, wabenförmige Struktur aufweist. Die Größe einer Einheits- respektive Elementarzelle dieses sich zyklisch wiederholenden Musters ändert sich entlang der Querrichtung des Signalleiters 21, also entlang der durch den Pfeil 23 angedeuteten Richtung. Zur Generierung dieser Struktur wurde der Signalleiter 21 mittels eines 3D-Druckers hergestellt. Konkret ist vorgesehen, dass bezüglich der Funksignale Frequenzen von etwa 60 GHz verwendet werden, wobei die Größe der Einheits- respektive Elementarzelle der Wabenstruktur maximal einem Zehntel der zugehörigen Wellenlänge entspricht, sodass eine kontinuierliche und mithin sprungfreie Änderung der Permittivität realisiert wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.

## Patentansprüche

1. Verfahren zur Durchführung einer Datenübertragung von einem rotierenden Abschnitt (4) eines medizinischen Bildgebungsgeräts (2) zu einem stationären Abschnitt (3) des medizinischen Bildgebungsgeräts (2), wobei die Datenübertragung mittels an dem rotierenden Abschnitt (4) angeordneten Funksendern (11, 13, 17, 18) und an dem stationären Abschnitt (3) angeordneten Funkempfängern (12, 15, 19, 20) erfolgt, **dadurch gekennzeichnet, dass** mehrere separate Übertragungspaare, die jeweils einen der Funksender (11) und einen der Funkempfänger (12) umfassen, gebildet werden, wobei bei den Übertragungspaaren jeweils Funksignale von dem Funksender (11) zu dem Funkempfänger (12) übertragen werden und eine die Funksignale betreffende Hauptabstrahlrichtung des Funksenders (11) zu der Richtung des Funkempfängers (12) nachgeführt wird.

2. Verfahren nach Anspruch 1, **dadurch gekennzeichnet, dass** die Übertragungspaare zu Beginn der Datenübertragung dadurch vorgegeben und/oder während der Durchführung der Datenübertragung dadurch aktualisiert werden, dass dem Funksender (11) des jeweiligen Übertragungspaars derjenige der Funkempfänger (12) zugeordnet wird, der aktuell den kürzesten Abstand zu diesem Funksender (11) aufweist und/oder der mit dem jeweiligen Funksender (11) aktuell über eine direkte Sichtlinie verbindbar ist.

3. Verfahren nach Anspruch 1 oder 2, **dadurch gekennzeichnet, dass** wenigstens ein die Qualität der Übertragung der Funksignale bei dem jeweiligen Übertragungspaar betreffender Qualitätsparameter und/oder wenigstens ein die Rotation des rotierenden Abschnitts (4) betreffender Rotationsparameter ermittelt wird, wobei ein Aktualisierungszeitpunkt, an dem die oder eine Aktualisierung des wenigstens einen Übertragungspaars erfolgt, anhand des wenigstens einen Qualitätsparameters und/oder des wenigstens einen Rotationsparameters vorgegeben wird.

4. Verfahren nach einem der vorangehenden Ansprüche, **dadurch gekennzeichnet, dass** während eines kompletten Umlaufs des rotierenden Abschnitts jeder der Funksender mit jedem der Funkempfänger eines der Übertragungspaare bildet.

5. Verfahren zur Durchführung einer Datenübertragung von einem rotierenden Abschnitt (4) eines medizinischen Bildgebungsgeräts (2) zu einem stationären Abschnitt (3) des medizinischen Bildgebungsgeräts (2), wobei die Datenübertragung mittels an dem rotierenden Abschnitt (4) angeordneten Funksendern (11, 13, 17, 18) und an dem stationären Abschnitt (3) angeordneten Funkempfängern (12, 15, 19, 20) erfolgt, **dadurch gekennzeichnet, dass** mehrere separate Übertragungspaare, die jeweils einen der Funksender (13, 17, 18) und einen der Funkempfänger (15, 19, 20) umfassen, gebildet werden, wobei bei den Übertragungspaaren jeweils Funksignale von dem Funksender (13, 17, 18) zu dem Funkempfänger (15, 19, 20) übertragen werden, wobei zu den mittels der Funkempfänger (15, 19, 20) empfangenen Funksignalen jeweils wenigstens eine Zuordnungsinformation ermittelt wird, die denjenigen Funksender (13, 17, 18) beschreibt, der dieses Funksignal generiert hat, wobei bei den Übertragungspaaren jeweils mittels der wenigstens einen Zuordnungsinformation diejenigen von dem Funkempfänger (15, 19, 20) empfangenen Funkdaten ausgefiltert werden, die nicht von dem Funksender (13, 17, 18) dieses Übertragungspaars generiert wurden.

6. Verfahren nach Anspruch 5, **dadurch gekennzeichnet, dass** die Zuordnungsinformation oder eine der Zuordnungsinformationen
- eine Distanzinformation ist, die eine Distanz zwischen dem jeweiligen Funkempfänger (12, 19, 20) und dem Funksender (13, 17, 18), mittels dem das jeweilige Funksignal generiert wurde, betrifft, oder
- eine Polarisationsinformation ist, die eine Polarisation des jeweiligen Funksignals betrifft, oder
- eine Frequenzinformation ist, die eine Frequenz oder ein Frequenzband des jeweiligen Funksignals betrifft.

7. Verfahren nach Anspruch 6, **dadurch gekennzeichnet, dass** die Zuordnungsinformation oder eine der Zuordnungsinformationen die Frequenzinformation ist, wobei die Funksender (13, 17, 18) jeweils einer von mehreren Funksendergruppen (14) zugeordnet sind, wobei die Funksender (13, 17, 18) einer der Funksendergruppen (14) jeweils Funksignale im gleichen Frequenzbereich generieren, wobei die Funksender (13, 17, 18) unterschiedlicher Funksendergruppen (14) jeweils Funksignale in verschiedenen Frequenzbereichen generieren, wobei die Funksender (13, 17, 18) der Funksendergruppen (14) jeweils gruppiert und beabstandet zu den Funksendern (13, 17, 18) der wenigstens einen anderen Funksendergruppe (14) an dem rotierenden Abschnitt (4) angeordnet sind.

8. Verfahren nach einem der Ansprüche 5 bis 7, **dadurch gekennzeichnet, dass** die Funksender (13, 17, 18) jeweils wenigstens eine bezüglich des rotierenden Abschnitts (4) feste Hauptabstrahlrichtung der Funksignale aufweisen.

9. Verfahren nach Anspruch 8, **dadurch gekennzeichnet, dass** die Hauptabstrahlrichtung bei wenigstens einem der Funksender (13, 17, 18) und eine Hauptempfangsrichtung bei wenigstens einem der Funkempfänger (15, 19, 20), die eine bezüglich des Empfangs der Funksignale sensitive Raumrichtung betrifft, entlang und/oder entgegen einer Rotationsrichtung betreffend die Rotation des rotierenden Abschnitts (4) weist.

10. Verfahren nach Anspruch 9, **dadurch gekennzeichnet, dass** die Funksender (13, 17, 18) jeweils einer vorwärts gerichteten Funksendermenge oder einer rückwärts gerichteten Funksendermenge zugeordnet sind, wobei für die Funksender (13, 17, 18) der vorwärts gerichteten Funksendermenge vorgesehen ist, dass deren Hauptabstrahlrichtungen entlang der Rotationsrichtung weisen, wobei für die Funksender (13, 17, 18) der rückwärts gerichteten Funksendermenge vorgesehen ist, dass deren Hauptabstrahlrichtungen entgegen der Rotationsrichtung weisen, wobei die Funkempfänger (15, 19, 20) jeweils einer vorwärts gerichteten Funkempfängermenge oder einer rückwärts gerichteten Funkempfängermenge zugeordnet sind, wobei für die Funkempfänger (15, 19, 20) der vorwärts gerichteten Funkempfängermenge vorgesehen ist, dass deren Hauptempfangsrichtungen entlang der Rotationsrichtung weisen, wobei für die Funkempfänger (15, 19, 20) der rückwärts gerichteten Funkempfängermenge vorgesehen ist, dass deren Hauptempfangsrichtungen entgegen der Rotationsrichtung weisen.

11. Verfahren zur Durchführung einer Datenübertragung von einem rotierenden Abschnitt (4) eines medizinischen Bildgebungsgeräts (2) zu einem stationären Abschnitt (3) des medizinischen Bildgebungsgeräts (2), wobei die Datenübertragung mittels an dem rotierenden Abschnitt (4) angeordneten Funksendern (11, 13, 17, 18) und an dem stationären Abschnitt (3) angeordneten Funkempfängern (12, 15, 19, 20) erfolgt, **dadurch gekennzeichnet, dass** von den Funksendern (13, 17, 18) zu den Funkempfängern (15, 19, 20) übertragene Funksignale in wenigstens einen zu den Funkempfängern (15, 19, 20) führenden und aus einem dielektrischen Material bestehenden Signalleiter (21) eingekoppelt werden, wobei der Signalleiter (21) eine sich quer zu einer Erstreckungsrichtung ändernde Permittivität aufweist.

12. Verfahren nach Anspruch 11, **dadurch gekennzeichnet, dass** der Signalleiter (21) aus einem Kunststoff, insbesondere aus Polystyrol und/oder Polyethylen und/oder Polypropylen und/oder Polytetrafluoräthylen, besteht.

13. Verfahren nach Anspruch 11 oder 12, **dadurch gekennzeichnet, dass** der Signalleiter (21) eine sich ändernde geometrische, insbesondere wabenförmige, Struktur aufweist, wobei die sich ändernde Permittivität mittels der sich ändernden geometrischen Struktur realisiert ist.

14. Verfahren nach einem der vorangehenden Ansprüche, **dadurch gekennzeichnet, dass** der stationäre Abschnitt (3) einen zylindrischen Aufnahmebereich aufweist, in dem der rotierende Abschnitt (4) angeordnet ist, wobei die Funksender (11, 13, 17, 18) entlang eines Umfangs, insbesondere entlang eines Außenumfangs, des rotierenden Abschnitts (4) angeordnet sind, wobei die Funkempfänger (12, 15, 19, 20) entlang eines Umfangs, insbesondere entlang eines Innenumfangs, des Aufnahmebereichs angeordnet sind.

15. Medizinische Bildgebungseinrichtung (1), umfassend ein medizinisches Bildgebungsgerät (2), insbesondere ein Computertomographiegerät, mit einem rotierenden Abschnitt (4) und einem stationären Abschnitt (3), wobei an dem rotierenden Abschnitt (4) mehrere Funksender (11, 13, 17, 18) und an dem stationären Abschnitt (3) mehrere Funkempfänger (12, 15, 19, 20) angeordnet sind, wobei mittels der Funksender (11, 13, 17, 18) und den Funkempfängern (12, 15, 19, 20) eine Datenübertragung von dem rotierenden Abschnitt (4) zu dem stationären Abschnitt (3) durchführbar ist,
**dadurch gekennzeichnet,**
- **dass** eine Steuerungseinrichtung (10) vorgesehen ist, die dazu eingerichtet ist, Steuersignale zur Steuerung des Betriebs der Funksender (11) und der Funkempfänger (12) derart zu generieren, dass mehrere separate Übertragungspaare, die jeweils einen der Funksender (11) und einen der Funkempfänger (12) umfassen, gebildet werden, wobei bei den Übertragungspaaren jeweils Funksignale von dem Funksender (11) zu dem Funkempfänger (12) übertragen werden und eine die Funksignale betreffende Hauptabstrahlrichtung des Funksenders (11) zu der Richtung des Funkempfängers (12) nachgeführt wird, oder
- **dass** eine Steuerungseinrichtung (10) vorgesehen ist, die dazu eingerichtet ist, Steuersignale zur Steuerung des Betriebs der Funksender (11) und der Funkempfänger (12) derart zu generieren, dass mehrere separate Übertragungspaare, die jeweils einen der Funksender (13, 17, 18) und einen der Funkempfänger (15, 19, 20) umfassen, gebildet werden, wobei bei den Übertragungspaaren jeweils Funksignale von dem Funksender (13, 17, 18) zu dem Funkempfänger (15, 19, 20) übertragen werden, wobei zu den mittels der Funkempfänger (15, 19, 20) empfangenen Funksignalen jeweils wenigstens eine Zuordnungsinformation ermittelt wird, die denjenigen Funksender (13, 17, 18) beschreibt, der dieses Funksignal generiert hat, wobei bei den Übertragungspaaren jeweils mittels der wenigstens einen Zuordnungsinformation diejenigen von dem Funkempfänger (15, 19, 20) empfangenen Funkdaten ausgefiltert werden, die nicht von dem Funksender (13, 17, 18) dieses Übertragungspaars generiert wurden, oder
- **dass** von den Funksendern (13, 17, 18) zu den Funkempfängern (15, 19, 20) übertragene Funksignale in wenigstens einen zu den Funkempfängern (15, 19, 20) führenden und aus einem dielektrischen Material bestehenden Signalleiter (21) einkoppelbar sind, wobei der Signalleiter (21) eine sich quer zu einer Erstreckungsrichtung ändernde Permittivität aufweist.
